# EUROPEAN PATENT APPLICATION

(11) **EP 4 803 170 A2**
(43) Date of publication of application: **09.09.2026**
(21) Application number: 26175396.6
(22) Date of filing: 28.09.2022
(51) Int. Cl.: B01D 15/34

(54) **AN IMPROVED PROCESS FOR PURIFICATION OF PROTEIN**

(30) Priority: 28.09.2021 IN 202121043959; 28.09.2021 IN 202121043962
(62) Document number(s) of the earlier application(s) in accordance with Art. 76 EPC: 22875292.9 / 4 408 856
(71) Applicant: Kashiv Biosciences, LLC, Piscataway, NJ 08854 (US)
(72) Inventor: NARAYAN, Om, Gujarat Ahmedabad 380051 (IN); GUPTA, Tarun Kumar, Gujarat Ahmedabad 380058 (IN); THAKKAR, Mayankkumar, Gujarat Ahmedabad 382481 (IN); UPADHYAY, Roshan Ganeshlal, Gujarat Ahmedabad 380061 (IN)
(74) Representative: Harrison IP Limited

(57) **Abstract**

The present invention relates to purification process of pharmacologically active IgG1 containing protein comprising at least affinity chromatography followed by mixed-mode chromatography. The present invention providescytotoxic T-lymphocyte-associated 4-immunoglobulin (CTLA4-Ig) fusion protein by using at least affinity chromatography, mixed-mode chromatography and optionally one or more suitable purification steps that provides purified composition of the fusion protein, substantially free of impurities selected from Pre-Peak, product and process related impurities. Further, the present invention provides highly purified CTLA4-Ig fusion protein with reduced heterogeneity.

## Description

### Field of the Invention

The present invention relates to purification process of pharmacologically active IgG1 containing protein comprising at least affinity chromatography followed by mixed-mode chromatography. The present invention provides cytotoxic T-lymphocyte-associated 4-immunoglobulin (CTLA4-Ig) fusion protein by using at least affinity chromatography, mixed-mode chromatography and optionally one or more suitable purification steps that provides purified composition of the fusion protein, substantially free of impurities selected from Pre-Peak, product and process related impurities. Further, the present invention provides highly purified CTLA4-Ig fusion protein with reduced heterogeneity.

### Background of the invention

Purification at large scale of proteins, e.g., therapeutic proteins including antibodies or fusion protein, is an increasingly important consideration for biopharmaceutical industries. With the advancement of biotechnology, fusion proteins are considered a promising therapeutic area for the treatment of diseases. However, fusion proteins are complex in nature as made of fusion of receptor (natural or modified) and immunoglobulin constant region (Fc including with or without hinge region or modified Fc). As per the complexity of the fusion proteins and its challenging purification process gives a motivation to minimize the impurities from fusion protein that proportionally affect the stability and functional efficacy. The recent advances in mammalian cell culture processes have significantly increased product titers as well as process and product-related impurities. Aggregation, charge variants, high molecular weight (HMW), low molecular weight (LMW) like impurities with the fusion protein has been a major problem that has been associated with a change in protein structure and being a hurdle in various upstream and downstream purification processes. The Fc-fusion proteins have elevated levels of aggregates, high molecular weight species (HMWs; up to 20%) and low molecular weight species (LMWs; up to 20%) within the product species. Glycosylation of proteins and the subsequent processing of the added carbohydrates can affect protein folding and structure, protein stability, including protein half-life, and functional properties of a protein. Desired glycosylation can be obtained through clone and upstream process. These impurities generated during the manufacturing process leads to decreased product yield, peak broadening, and reduce or loss of activity of molecule. The improvement or a step forward in Fc-fusion protein processing is crucially needed as conventional chromatography column requires various conditioning parameters of protein before loading onto chromatography column, lacking effective purification process to remove product and process related impurities, that directly impacts process time, cost, and labor effort in protein handling. The present invention provides a purification process by selecting and arranging the affinity chromatography, mixed-mode chromatography and optionally one or more suitable purification stepsto remove impurities associated with the fusion protein. The process reduces or removes at least one impurity selected from Pre-Peak, HMW, LMW, HCP, and undesired glycan.

There is a present need for methods of producing and purifying a fusion protein of interest in sufficiently pure form to be suitable for pharmaceutical use.

### Summary of the Invention

In an embodiment, the invention provides a chromatography process to produce a composition enriched with fusion protein of interest and substantially reduced impurities selected from acidic variants, basic variants, high molecular weight (HMW), low molecular weight (LMW), pre-peak or aggregates.

In an embodiment, the invention provides a chromatography process to produce a composition enriched with fusion protein of interest and substantially reduced amount of acidic variant impurities.

In an embodiment, the invention provides a chromatography process to produce a composition enriched with fusion protein of interest and substantially reduced basic variant impurities.

In an embodiment, the invention provides a chromatography process to produce a composition enriched with fusion protein of interest and substantially reduced high molecular weight (HMW) impurities.

In an embodiment, the invention provides a chromatography process to produce a composition enriched with fusion protein of interest and substantially reduced low molecular weight (LMW) impurities.

In an embodiment, the invention provides a chromatography process to produce a composition enriched with fusion protein of interest and substantially reduced level of undesired glycans.

In an embodiment, the invention provides a chromatography process to produce a composition enriched with fusion protein of interest and substantially reduced pre-peak impurities or aggregates. In an embodiment the invention provides a mixed-mode (MMC) chromatography to produce a composition enriched with fusion protein of interest and substantially reduced pre-peak impurities or aggregates.

In an embodiment, a method for purifying a Fc-fusion protein from a protein sample comprising Fc-fusion protein and Pre-peak impurity:
a) contacting the protein sample onto mixed-mode chromatography column;
b) optionally perform washing the mixed-mode chromatography column;
c) eluting the fusion protein from a mixed-mode chromatography column; wherein the eluted fusion protein comprises reduce amount of Pre-peak impurity.

In one aspect of such embodiment, the pre-peak impurity reduced by at least more than about 90%, about 91%, about 92%, about 93%, about 94%, about 95%, about 96%, about 97%, about 98%, about 99%. In an embodiment, the pre-peak impurity is below quantifiable limit.

In an embodiment, a method for purifying a Fc-fusion protein from a protein sample comprising:
a) contacting the protein sample onto mixed-mode chromatography column;
b) washing the mixed-mode chromatography column with buffer comprising a suitable additive;
c) eluting the fusion protein from a mixed-mode chromatography column with reduced amount of impurities;
wherein, the suitable additive is arginine or arginine derivatives.

In an embodiment, the concentration of arginine or arginine derivatives is selected from about 20mM to about 100mM.

In another embodiment, the concentration of arginine or arginine derivative is selected from about 60mM to about 80mM.

In an embodiment, a method for purifying a Fc-fusion protein from a protein sample comprising:
a) contacting the protein sample onto mixed-mode chromatography column;
b) washing the mixed-mode chromatography column with a suitable buffer comprising suitable additive;
c) eluting the fusion protein from a mixed-mode chromatography column;
wherein the elution buffer comprises arginine or arginine derivatives concentration selected from below 600mM, preferably 300mM.

In an embodiment, the eluted fusion protein from mixed-mode chromatography column comprises reduced amount impurity compared to elution without using arginine or arginine derivatives in wash and elution buffers.

In an embodiment, a process for purification of IgG1 containing protein comprising:
a) collecting the IgG1 containing protein from the suitable mammalian expression system and the impurities;
b) contacting the IgG1 containing protein on to a affinity chromatography;
c) eluting the IgG1 containing protein;
d) contacting the eluted IgG1 containing protein onto mixed-mode chromatography, optionally followed by other suitable purification steps, wherein mixed-mode chromatography is performed in bind-elute mode.

In an embodiment, the process bind-elute mode comprises: (i) loading the eluted IgG1 protein from Affinity chromatography at suitable pH and/or conductivity for binding (ii) performing washing at suitable pH and/or conductivity (iii) eluting the IgG1 protein at suitable pH and/or conductivity.

In an embodiment, the loading is performed at suitable pH selected from about 6.5 to about 7.5.

In an embodiment, the loading is performed at suitable conductivity selected from about 3 mS/cm to about 5 mS/cm.

In an embodiment, the invention provides an affinity chromatography and mixed mode chromatography to produce a composition comprising enriched CTLA-4 IgG1 fusion protein and significantly reduced high molecular weight (HMW) impurities selected from about 10%, about 9%, about 8%, about 7%, about 6%, about 5%, about 4%, about 3%, about 2%, about 1%, about 0.5% or less HMW.

In one aspect of this embodiment, the low HMW composition comprises about 0.9% or less HMW, about 0.8% or less HMW, about 0.7% or less HMW, about 0.6% or less HMW, about 0.5% or less HMW, about 0.4% or less HMW, about 0.3% or less HMW, about 0.2% or less HMW, about 0.1% or less HMW.

In an embodiment, the invention provides an affinity chromatography and mixed mode chromatography to produce a composition comprising enriched CTLA-4 IgG1 fusion protein and reduced LMW impurities selected from about 50%, about 40%, about 30%, about 20%, about 10%, about 9%, about 8%, about 7%, about 6%, about 5%, about 4%, about 3%, about 2%, about 1%, about 0.5%, about 0.4%, about 0.3%, about 0.2%, or less LMW or below quantifiable limit.

In an embodiment, a method for purifying a Fc-fusion protein from a protein sample comprising Fc-fusion protein and at least Pre-peak impurity:
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted Fc-fusion protein is substantially free of Pre-peak impurities.

In one aspect of such embodiment, the pre-peak impurity reduced by at least more than 90%, 91%, 92%, 93%, 94%, 95%, 96%, 97%, 98%, 99%. In an embodiment the pre-peak impurity is below quantifiable limit.

In an embodiment, a method for purifying a Fc-fusion protein from a protein sample comprising Fc-fusion protein and at least Low molecular weight (LMW):
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of low molecular weight (LMW) impurities.

In an embodiment, the invention utilizes chromatography column to produce a Fc-fusion protein composition comprising about 0.5% or less LMW, about 0.4% or less LMW, about 0.3% or less LMW, about 0.2% or less LMW, about 0.1% or less LMW.

In an embodiment, a method for purifying a Fc-fusion protein from a protein sample comprising Fc-fusion protein and at least High molecular weight (HMW):
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of high molecular weight (HMW) impurities.

In an embodiment, the Fc-fusion protein composition comprising about 0.5% or less HMW, about 0.4% or less HMW, about 0.3% or less HMW, about 0.2% or less HMW, about 0.1% or less HMW.

In an embodiment, a method for purifying a Fc-fusion protein from a protein sample comprising Fc-fusion protein and undesired glycans:
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of undesired glycans.

In such embodiment, undesired glycans selected from high mannose and afucosylated variant are reduced by 76.54% and about 24.35% respectively.

In such embodiment, high mannose reduced by less than 80% preferably less than 77%.

In such embodiment, afucosylated variants are reduced by less than 30% preferably less than 25%. In one aspect of such embodiment, the all-column chromatography is performed in bind elute mode.

In one aspect of such embodiment, all column chromatography uses same buffer.

In one aspect of such embodiment, viral inactivation is performed with a suitable detergent for about 60 minutes.

In one aspect of such embodiment, pH-based virus inactivation is not performed in eluate of affinity chromatography.

In an embodiment, the invention is related to the process for the purification of the fusion protein which is free from at least one impurity selected from Host cell proteins (HCP) comprising:
a) collecting the first protein sample/mixture from the suitable mammalian expression system comprising fusion protein and impurities;
b) contacting the protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein the eluted fusion protein is substantially free of host cell proteins (HCP) impurities.

In an embodiment, the fusion protein comprises reduced HCP impurities at drug substance level after purification selected from about 10 ng/mg or less, 9 ng/mg or less, 8 ng/mg or less,7 ng/mg or less,6 ng/mg or less,5 ng/mg or less,4 ng/mg or less,3 ng/mg or less,2 ng/mg or less,1 ng/mg or less.

In an embodiment, the invention provides an affinity chromatography and mixed mode chromatography to produce a composition comprising enriched fusion protein and significantly reduced high molecular weight (HMW) impurities about 10%, about 9%, about 8%, about 7%, about 6%, about 5%, about 4%, about 3%, about 2%, about 1%, about 0.5% or less.

In one aspect of this embodiment, the low HMW composition comprises about 0.5% or less HMW, about 0.4% or less HMW, about 0.3% or less HMW, about 0.2% or less HMW, about 0.1% or less.

In an embodiment, the invention provides an affinity chromatography and mixed mode chromatography to produce a composition comprising enriched fusion protein and substantially reduced LMW or fragments.

In such embodiment, LMW reduced by about 10%, about 9%, about 8%, about 7%, about 6%, about 5%, about 4%, about 3%, about 2%, about 1%, about 0.5%, or about 0.3% less.

In an embodiment, the low LMW composition comprises about 0.5% less LMW, about 0.4% or less LMW, about 0.3% or less LMW, about 0.2% or less LMW, about 0.1% or less LMW or below quantifiable limit.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein the eluted CTLA-4 IgG1 fusion protein is substantially free of LMW and HMW impurities.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the CTLA-4 IgG1 fusion protein from anion exchange chromatography column to form fourth protein mixture;
h) contacting the fourth protein mixture to hydrophobic interaction chromatography column;
i) eluting the CTLA-4 IgG1 fusion protein from hydrophobic interaction chromatography column to form fifth protein mixture;
wherein the eluted fusion protein is substantially free of Pre-peak, LMW and HMW impurities.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the CTLA-4 IgG1 fusion protein from anion exchange chromatography column to form fourth protein mixture;
wherein the eluted fusion protein is substantially free of Pre-peak, LMW and HMW impurities.

In an embodiment, the invention provides an affinity chromatography, mixed mode chromatography and anion exchange chromatography to produce a composition comprising enriched CTLA-4 IgG1 fusion protein and significantly reduced high molecular weight (HMW) impurities selected from about 10%, about 9%, about 8%, about 7%, about 6%, about 5%, about 4%, about 3%, about 2%, about 1%, about 0.5% or less HMW.

In one aspect of this embodiment, the low HMW composition comprises about 0.9% or less HMW, about 0.8% or less HMW, about 0.7% or less HMW, about 0.6% or less HMW, about 0.5% or less HMW, about 0.4% or less HMW, about 0.3% or less HMW, about 0.2% or less HMW, about 0.1% or less HMW.

In an embodiment, the invention provides an affinity chromatography, mixed mode chromatography and anion exchange chromatography to produce a composition comprising enriched CTLA-4 IgG1 fusion protein and reduced LMW impurities selected from about 50%, about 40%, about 30%, about 20%, about 10%, about 9%, about 8%, about 7%, about 6%, about 5%, about 4%, about 3%, about 2%, about 1%, about 0.5%, about 0.4%, about 0.3%, about 0.2%, or less LMW or below quantifiable limit.

In an embodiment, the invention provides an affinity chromatography, mixed mode chromatography and anion exchange chromatography to produce a composition comprising enriched CTLA-4 IgG1 fusion protein and reduced undesired glycans. In such embodiment, undesired glycans are selected from high mannose are reduced by less than 80%, preferably 77%.

In such embodiment, undesired glycans selected from high mannose and afucosylated variants.

In such embodiment, high mannose reduced by less than 80%, preferably less than 77%.

In such embodiment, afucosylated variants are reduced by less than 30%, preferably less than 25%.

In such embodiment, the high mannose is reduced by about 95%, about 94%, about 93%, about 92%, about 91%, about 90%, about 89%, about 88%, about 87%, about 86%, about 85%, about 84%, about 83%, about 82%, about 81%, about 80%, about 79%, about 78%, about 77%, about 76%, and about 75%.

In an embodiment the present invention provides a large-scale purification process of CTLA4-Fc fusion protein to maintain the quality and/or quantity of purified protein composition.

### Detail Description of Figures

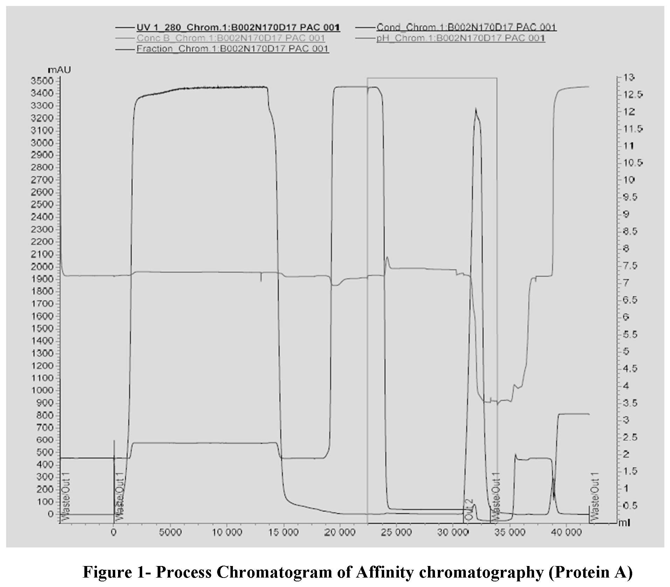

**Figure 1**- Process Chromatogram of Affinity chromatography (Protein A).

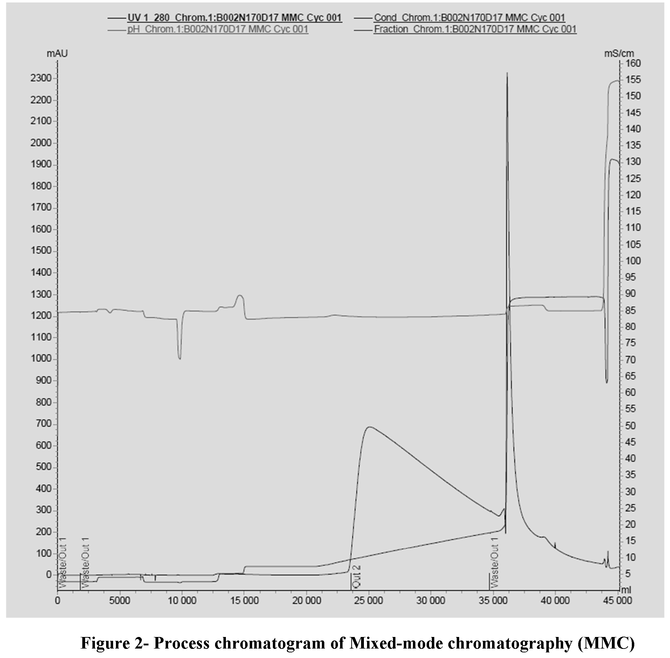

**Figure 2****-** Process chromatogram of Mixed-mode chromatography (MMC).

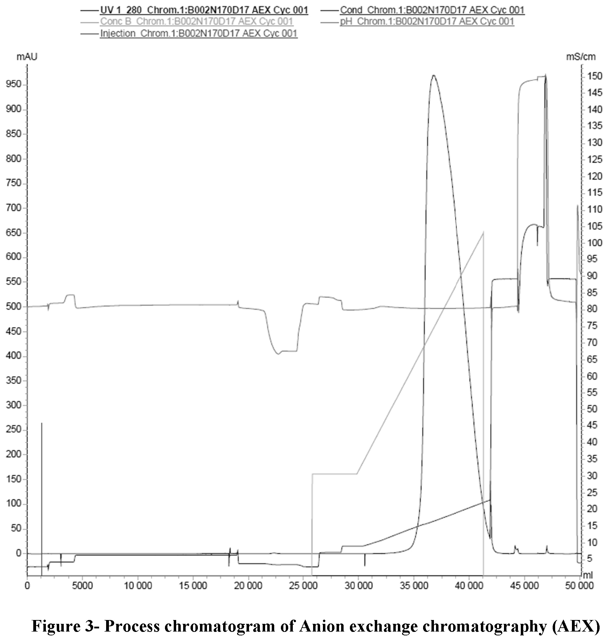

**Figure 3****-** Process chromatogram of Anion exchange chromatography (AEX).

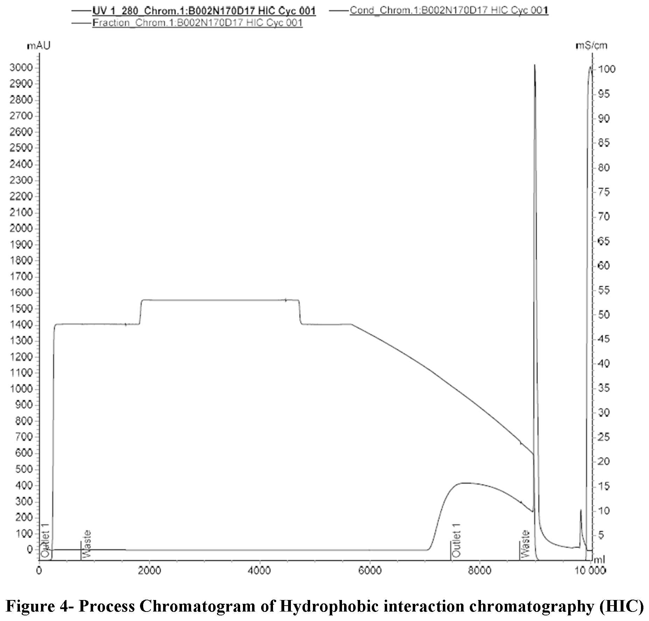

**Figure 4****-** Process Chromatogram of Hydrophobic interaction chromatography (HIC).

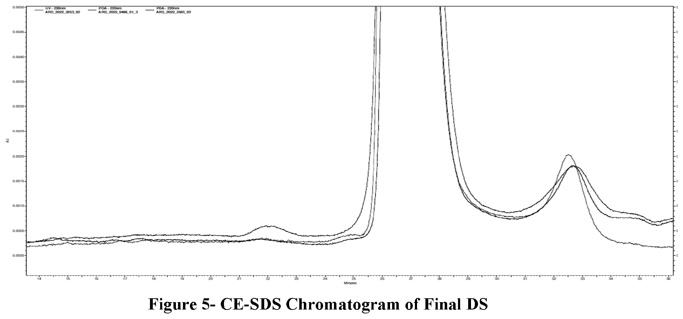

**Figure 5****-** CE-SDS Chromatogram of Final DS.

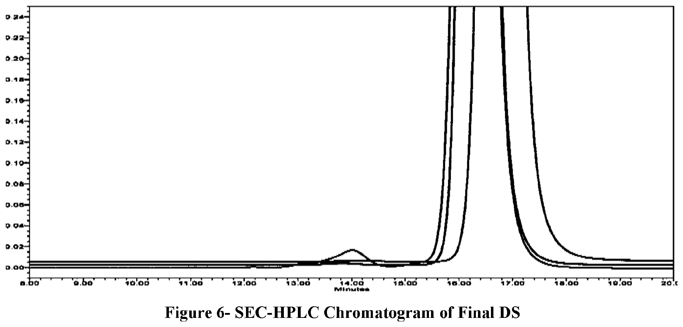

**Figure 6****-** SEC-HPLC Chromatogram of Final DS.

### Detail Description of Invention

As used herein the term "column" or "resin" or "chromatographic resin or chromatographic column" are interchangeable.

The term "comprises" or "comprising" is used in the present description, it does not exclude other elements or steps. For purpose of the present invention, the term "consisting of' is considered to be an optional embodiment, of the term "comprising of'. If hereinafter a group is defined to comprise at least a certain number of embodiments, this is also to be understood to disclose a group which optionally consists only of these embodiments. As used throughout the specification and in the appended claims, the singular forms "a," "an," and "the" include the plural reference unless the context clearly dictates otherwise.

The term "about", as used herein, is intended to refer to ranges of approximately 10-20% greater than or less than the referenced value. In certain circumstances, one of skill in the art will recognize that, due to the nature of the referenced value, the term "about" can mean more or less than a 10-20% deviation from that value.

The term "clarified harvest cell culture fluid" or "HCCF" or "harvest cell culture fluid" used herein are interchangeable refers to the protein mixture obtained from mammalian cell culture containing protein of interest along with other impurities.

The term "affinity chromatography" refers to chromatography processes of separating biochemical mixtures based on a highly specific interaction between e.g., antigen and antibody, enzyme and substrate, receptor and ligand, or protein and nucleic acid. Examples of such chromatographic resins include, but are not limited to Protein A resin, Protein G resin, Protein L resin, immobilized metal ion affinity chromatography etc.

The term "Protein A affinity chromatography" use for the separation or purification of substances and/or particles using protein A, where the protein A is generally immobilized on a solid phase.

Protein A is a 40-60 kD cell wall protein originally found in Staphylococcus aureus. The binding of fusion protein to protein A resin is highly specific. Protein A affinity chromatography columns for use in protein A affinity chromatography herein include, but are not limited to, Protein A immobilized on a polyvinylether solid phase, e.g., the Eshmuno^{®} columns (Merck, Darmstadt, Germany), Protein A immobilized on a pore glass matrix, e.g., the ProSep^{®} columns (Merck, Darmstadt, Germany) Protein A immobilized on an agarose solid phase, for instance the MABSELECT^{™} SuRe^{™} columns (GE Healthcare, Uppsala, Sweden).

The term "MabSelect SuRe LX" used herein is a protein A affinity resin with very high dynamic binding capacity at extended residence times. The ligand is alkali-stabilized protein A-derived (E. coli) shows alkali tolerance, high capacity and low ligand leakage in combination with the rigid base matrix.

The term "mixed mode chromatography" or "MMC" refers to chromatography a combination of two or more chemical interactions within in a solid support. The examples of chemical interactions that can be combined in mixed mode supports include but are not limited to cation exchange, anion exchange, hydrophobic interaction, hydrophilic interaction, hydrogen bonding, pi-pi bonding, and metal affinity.

The term "Nuvia aPrime 4A" used herein is a hydrophobic anion exchange resin designed with a distinct balance of modes optimized for biomolecule interactions. The resin's ligand density and hydrophobicity are engineered to facilitate selective and reversible binding of target molecules for high purity and yield. Nuvia aPrime offers a wide design space and straightforward method development to purify even the most complex molecules The resin has aromatic hydrophobic anion exchanger chemistry.

The term "anion exchange chromatography" or "anion exchange column" or "AEX" used herein is a form of ion exchange chromatography (IEX), which is used to separate molecules based on their net surface charge. Anion exchange chromatography, more specifically, uses a positively charged ion exchange resin with an affinity for molecules having net negative surface charges. Anion exchange chromatography is used both for preparative and analytical purposes and can separate a large range of molecules, from amino acids and nucleotides to large proteins. Here, we focus on the preparative anion exchange chromatography of proteins.

As used herein the term "bind and elute mode" or "B/E" refers to purification process wherein the fusion protein of interest binds to chromatography resin. At least about 90% fusion protein of interest bind to chromatographic resin. At least about 60% or about 70% or about 80% fusion protein of interest binds to chromatographic resin. However, process and product related impurities does not bind the chromatographic resin. At least about 50% process and product related impurities does not bind to chromatographic resin. At least about 60% or about 70% or about 80% process and product related impurities does not bind to chromatographic resin.

In an embodiment, the anion exchange is strong anion exchange. The term "POROS XQ" used herein is a Thermo Scientific POROS XQ Strong Anion Exchange Resin are designed for charge based chromatographic separations of biomolecules including recombinant proteins, monoclonal antibodies. POROS XQ has high and consistent protein capacity across a broad range of salt concentrations. The resin has quaternary amine groups.

The term "hydrophobic interaction chromatography" or "HIC" refers to a column containing a stationary phase or resin and a mobile or solution phase in which the hydrophobic interaction between a protein and hydrophobic groups on the matrix serves as the basis for separating a protein from impurities including fragments and aggregates of the subject protein of interest, other proteins or other protein fragments and other contaminants such as cell debris, or residual impurities from other purification steps. The stationary phase comprises a base matrix or support such as a crosslinked agarose, silica or synthetic copolymer material to which hydrophobic ligands are attached. HIC relies on separation of proteins on the basis of hydrophobic interactions between non-polar regions on the surface of proteins and insoluble, immobilized hydrophobic groups on the matrix. The absorption increases with high salt concentration in the mobile phase and the elution is achieved by decreasing the salt concentration of the eluant.

The term "Poros Benzyl" used herein is Hydrophobic Interaction Chromatography (HIC) resin engineered for improved impurity removal during large-scale downstream purification of biomolecules and its capacity maintains excellent pressure flow characteristics, helps increase in productivity. The resin has POROS base bead with aromatic hydrophobic benzyl ligand, suited for purification.

The term "viral reduction/inactivation", as used herein, is intended to refer to a decrease in the number of viral particles in a particular sample ("reduction"), as well as a decrease in the activity, for example, but not limited to, the infectivity or ability to replicate, of viral particles in a particular sample ("inactivation").

The term used "pre-peak" demonstrates the analysis of the product related variant. The peak with the shortest retention time arising just before the main peak or monomer which contains product related impurities. Pre-peak may include but not limited to misfolded and aggregates of CTLA4-Ig.

The term "buffer" or "suitable buffer" refers to a solution that can resist pH change upon the addition of an acidic or basic components. It is able to neutralize small amounts of added acid or base, thus maintaining the pH of the solution relatively stable. This is important for processes and/or reactions which require specific and stable pH ranges. Buffer solutions have a working pH range and capacity which dictate how much acid/base can be neutralized before pH changes, and the amount by which it will change.

The term used herein "Buffer B" or "Elution buffer B" in mixed mode chromatography are interchangeable. For a non-limiting example, it refers to the buffer solution of 20mM sodium phosphate, 300 mM L-Arginine HCl at pH 7.2±0.2, conductivity 21.0±3.0 mS/cm.

The term used herein "Buffer B" or "Elution buffer B" in anion exchange chromatography are interchangeable. For a non-limiting example, it refers to the buffer solution of 20mM sodium phosphate, 300 mM sodium chloride at pH 7.2±0.2, conductivity 30.0±3.0 mS/cm.

The term used herein "Buffer B" or "Elution buffer B" in hydrophobic interaction chromatography are interchangeable. For a non-limiting example, it refers to the buffer solution of 20mM sodium phosphate at pH 7.2±0.2, conductivity 2.7±0.3 mS/cm. The term "neutralization" used herein refers to bringing back the acidic pH of the protein A eluate to a neutral pH by using basic solution.

The term used "aggregates" are classified based on types of interactions and solubility. Soluble aggregates are invisible particles and cannot be removed with a filter. Insoluble aggregates can be removed by filtration and are often visible to the human eye. Both types of aggregates cause problems in biopharma development. Covalent aggregates arise from the formation of a covalent bond between multiple monomers of a given peptide. Disulfide bond formation of free thiols is a common mechanism for covalent aggregation. Oxidation of tyrosine residues can lead to formation of bityrosine which often results in aggregation. Reversible protein aggregation typically results from weaker protein interactions they include dimers, trimers, multimers among others.

The term "Mannose" used herein refers to the high mannose.

The term "Afucosylation" used herein refers to the protein molecule do not have any fucose sugar units.

The term "undesired glycan" used herein refers to the N-glycan of the fusion protein that is beyond the acceptable range. In an embodiment, the acceptable N-glycan ranges includes high mannose, afucosylation, total sialylation which should be in desired range. The N-glycan profile of fusion protein should be in the acceptable range as mentioned above to comply the regulatory requirements.

The term "Host cell protein" or "HCP" refers to process-related protein impurities produced by the host organism during biotherapeutic manufacturing and production. HCPs may cause immunogenicity in individuals or reduce the potency, stability or overall effectiveness of a drug.

The term "Host cell DNA" or "HCD" used herein are interchangeable and refers to residual DNAs (rDNAs) are trace/low quantity of DNA originating from the organisms used in the production process of biopharmaceutical products.

The term used "high molecular weight" or "HMW" used herein are interchangeable and refers to product-related impurities that contribute to the size heterogeneity of fusion protein drug product. The formation of HMW species within a therapeutic fusion protein drug product as a result of protein aggregation can potentially compromise both drug efficacy and safety (e.g., eliciting unwanted immunogenic response). HMW is considered critical quality attribute that are routinely monitored during drug development and as part of release testing of purified drug product during manufacturing. In certain embodiment, the HMW relates to aggregates dimer, trimer or tetramer of CTLA4-Ig or monoclonal antibodies.

The term used "low molecular weight" or "LMW" used herein are interchangeable and refers to species which is a protein backbone-truncated fragments & considered as product-related impurities that contribute to the size heterogeneity of fusion protein. LMW species often have low or substantially reduced activity relative to the monomeric form of the fusion protein and can lead to immunogenicity or potentially impact pharmacokinetic properties in vivo. As a result, LMW species are considered critical quality attributes that are routinely monitored during drug development and as part of release testing of purified drug product during manufacturing. The LMW has molecular weight selected from less than about 80kDa or less, 75kDa or less, 70kDa or less, 65kDa or less, 60kDa or less, 55kDa or less, 50kDa or less, 45kDa or less, 40kDa or less, 35kDa or less, 30kDa or less, 25kDa or less, 20kDa or less, 15kDa or less, 10kDa or less.

The term "substantially pure fusion protein" used herein includes a fusion protein that is substantially free of impurity selected from product or process related impurities. The fusion protein is free of acidic variants, basic variants, low molecular weights and high molecular weights, substantially pure fusion protein has purity less than about 99% or less than about 98% or less than about 97% or less than about 95% or less than about 92% or less than about 90% or less than about 88% or less than about 85% or less than about 82% less than about 80% or less than about 75% or less than about 70% or less than about 65% or less than about 60% or less than about 50%.

The term "protein mixture" or "protein sample" used herein are interchangeable and refers to the solutions containing protein of interest along with other impurities.

The term "first protein mixture" or "first protein sample" or "HCCF" or "Harvest cell culture fluid" used herein are interchangeable which refers to the protein solution or mixture or sample obtained from mammalian cell culture before performing any chromatography column.

The term "second protein mixture" or "second protein solution" or "second protein sample" used herein are interchangeable which refers to the eluate of affinity chromatography.

The term "Neutralized Protein A Eluate" or "NPEL" used herein are interchangeable and it refers to the protein A eluate which has been further neutralized to a neutral pH. The term "Neutralized Protein A Elute" or "NPEL" also considering herein as "second protein mixture" or "second protein solution" or "second protein sample".

The term "third protein mixture" or "third protein solution" or "third protein sample" used herein are interchangeable which refers to the eluate of mixed mode chromatography (MMC).

The term "fourth protein mixture" or "fourth protein solution" or "fourth protein sample" used herein are interchangeable which refers to the eluate of anion exchange chromatography (AEX).

The term "fifth protein mixture" or "fifth protein solution" or "fifth protein sample" used herein are interchangeable which refers to the eluate of Hydrophobic Interaction chromatography (HIC).

The term "Residence time" refers to the amount of time a compound spends on the column after it has been injected. If a sample containing several compounds, each compound in the sample will spend a different amount of time on the column according to its chemical composition i.e., each will have a different retention time. Retention times are usually quoted in units of seconds or minutes.

The term "IgG1 containing protein" used herein refers to antibody or fusion protein. Fusion proteins or Fc-fusion protein are receptor fused with IgG ('ligand traps') have also been generated. TNF receptor 2-Fc (etanercept), rilonacept (Arcalyst - an IL-1 Trap), vascular endothelial growth factor trap (aflibercept), CTLA4-Fc fusion proteins (Abatacept and belatacept).

In another embodiment, antibodies are selected from IgG1, IgG2, IgG3, IgG4 and fusion proteins. In certain embodiments the antibodies are selected from Etanercept, Rituximab, Palivizumab, Infliximab, Trastuzumab, Alemtuzumab, Adalimumab, Ibritumomab tiuxetan, Omalizumab, Cetuximab ,Bevacizumab, Natalizumab, Eculizumab, Certolizumab pegol, Ustekinumab, Canakinumab, Golimumab, Ofatumumab, Tocilizumab, Denosumab, Belimumab, Ipilimumab, Brentuximab vedotin, Pertuzumab, Trastuzumab emtansine, Raxibacumab, Obinutuzumab, Siltuximab, Ramucimmab, Vedolizumab, Blinatumomab, Nivolumab, Pembrolizumab, Darucizumab, Necitumumab, Dinutuximab, Secukinumab, Mepolizumab, Alirocumab, Evolocumab, Daratumumab, Elotuzumab, Ixekizumab, Reslizumab, Olaratumab, Bezlotoxumab, Atezolizumab, Obiltoxaximab, Sarilumab, Ocrelizumab, Tildrakizumab, Romosozumab, Brolucizumab, and Crizanlizumab.

In an embodiment, the CTLA4-IgG1 or CTLA4-Fc is Abatacept or Belatacept.

The terms "CTLA4-Ig" or "CTLA4-Ig molecule" or "CTLA4-Fc molecule" used herein are interchangeable and refers to a protein molecule that comprises at least a polypeptide having a CTLA4 extracellular domain or portion thereof and an immunoglobulin constant region or portion thereof. The extracellular domain and the immunoglobulin constant region can be wild-type, or mutant or modified, and mammalian, including human or mouse. The polypeptide can further comprise additional protein domains. A CTLA4-Ig molecule can also refer to multimer forms of the polypeptide, such as dimers, tetramers, and hexamers. A CTLA4-Ig molecule is also capable of binding to CD80 and/or CD86. In an embodiment, the CTLA4-Ig is Abatacept.

The term "contacting" used herein refers to the loading of the protein mixture into the chromatography column.

The term "Drug Substance" or "DS" used herein are interchangeable and refers to an active ingredient intended to furnish pharmacological activity. The DS also refers to final protein mixture after all purification steps and substantially free from impurities.

In an embodiment, the present invention provides a combination of chromatographic methods selected from protein A chromatography, mixed mode chromatography, anion exchange chromatography and hydrophobic interaction chromatography to purify at least one impurity present in the mixture of CTLA-4 IgG1 fusion protein.

In an embodiment, the invention is related to a process for the purification of fusion protein by using Protein A (ProA) chromatography works in the bind and elute mode.

In another embodiment, the invention is related to a process for the purification of fusion protein followed by Mixed mode chromatography (MMC) works in the bind and elute mode.

In another embodiment, the invention is related to a process for the purification of fusion protein followed by Anion exchange chromatography (AEX) works in bind and elute mode.

In another embodiment, the invention is related to a process for the purification of fusion protein followed by Hydrophobic interaction chromatography (HIC) works in the bind and elute mode.

In yet another embodiment, the invention is related to the purification of fusion protein mixture comprising:
a) loading the fusion protein mixture onto first chromatography column;
b) optionally performing the washing;
c) eluting the fusion protein mixture from said first chromatography column;
d) optionally performing the filtration;
e) loading the fusion protein mixture obtained from step (C) or (D) onto mixed-mode chromatography;
f) optionally performing the washing;
g) eluting the fusion protein mixture from said mixed-mode chromatography;
wherein the first chromatography column is selected from Protein-A column, Hydrophobic interaction chromatography, anion exchange chromatography and cation exchange chromatography.

In an embodiment, a method for purifying a Fc-fusion protein from a protein sample comprising Fc-fusion protein and Pre-peak impurity:
a) contacting the protein sample onto mixed-mode chromatography column;
b) optionally perform washing the mixed-mode chromatography column;
c) eluting the fusion protein from a mixed-mode chromatography column;
wherein the eluted fusion protein comprises reduce amount of Pre-peak impurity.

In one aspect of such embodiment, the pre-peak impurity reduced by at least more than about 90%, about 91%, about 92%, about 93%, about 94%, about 95%, about 96%, about 97%, about 98%, about 99%. In an embodiment, the pre-peak impurity is below quantifiable limit.

In an embodiment, a process for purification of IgG1 containing protein comprising:
a) collecting the IgG1 containing protein from the suitable mammalian expression system and the impurities;
b) contacting the collecting the IgG1 containing protein Affinity chromatography;
c) eluting the IgG1 containing protein;
d) contacting the eluted IgG1 containing protein onto mixed-mode chromatography, optionally followed by other suitable purification steps, wherein mixed-mode chromatography is performed in bind-elute mode.

In one aspect of such embodiment, the pre-peak impurity reduced by at least more than about 90%, about 91%, about 92%, about 93%, about 94%, about 95%, about 96%, about 97%, about 98%, about 99%. In an embodiment the pre-peak impurity is below quantifiable limit.

In one aspect of this embodiment, the low HMW composition comprises about 0.5% or less HMW, about 0.4% or less HMW, about 0.3% or less HMW, about 0.2% or less HMW, about 0.1% or less HMW.

In an embodiment, the hydrophobic interaction chromatography reduces HMW and/or LMW by less than 50% preferably 70%.

In yet another embodiment, the invention is related to the purification of fusion protein by performing protein A chromatography, which is followed by mixed mode chromatography (MMC), which is followed by anion exchange chromatography (AEX), which is followed by hydrophobic interaction chromatography (HIC).

In an embodiment, the invention is related to the purification of fusion protein by performing protein A chromatography, which is followed by mixed mode chromatography (MMC), optionally further comprises one or more chromatography step.

In an embodiment, the invention is related to the purification of fusion protein by performing protein A chromatography, which is followed by mixed mode chromatography (MMC), optionally further comprises one or more chromatography step followed by ultrafiltration and diafiltration.

In an embodiment, the invention is related to the purification of fusion protein by performing protein A chromatography, which is followed by hydrophobic interaction chromatography (HIC), optionally further comprises one chromatography step.

In an embodiment, the invention is related to the purification of fusion protein by performing protein A chromatography, followed by optional viral inactivation or removal, which is followed by mixed mode chromatography (MMC), optionally further comprises one or more chromatography step.

In an embodiment, the invention is related to the purification of fusion protein by performing protein A chromatography, followed by mixed mode chromatography (MMC), optionally further followed by viral inactivation or removal, optionally further comprises one or more chromatography steps.

In one aspect of such embodiment, the third protein mixture is further incubated with detergent or surfactant for viral inactivation selected from about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 70 minutes, about 80 minutes.

In one aspect of such embodiment, the detergent or surfactant is selected from Triton X100 or Octo X100, sugar-based detergent *n*-dodecyl-β- D-maltoside, preferably Triton X100.

In an embodiment, the invention is related to the purification of fusion protein by performing protein A chromatography, followed by optional viral inactivation or removal, which is followed by hydrophobic interaction chromatography (HIC), optionally further comprises one chromatography step.

In an embodiment, the invention is related to the purification of fusion protein by performing protein A chromatography, followed by optional viral inactivation or removal, which is followed by mixed mode chromatography (MMC), optionally further comprises one or more chromatography step.

In an embodiment, the viral inactivation or removal performed using a surfactant or detergent which eliminates the use of harsh acid based viral inactivation or removal.

In an embodiment, the invention provides a chromatography process to produce a composition enriched with fusion protein of interest and substantially reduced impurities selected from acidic variants, basic variants, high molecular weight (HMW), low molecular weight (LMW), and prepeak or aggregates.

In an embodiment, the invention provides a chromatography process to produce a composition enriched with fusion protein of interest and substantially reduced amount of acidic variant impurities.

In an embodiment, the invention provides a chromatography process to produce a composition enriched with fusion protein of interest and substantially reduced basic variant impurities.

In an embodiment, the invention provides a chromatography process to produce a composition enriched with fusion protein of interest and substantially reduced high molecular weight (HMW) impurities.

In an embodiment, the invention provides a chromatography process to produce a composition enriched with fusion protein of interest and substantially reduced low molecular weight (LMW) impurities.

In an embodiment, the invention provides a chromatography process to produce a composition enriched with fusion protein of interest and substantially reduced level of undesired glycans.

In an embodiment, the invention provides a chromatography process to produce a composition enriched with fusion protein of interest and substantially reduced pre-peak impurities or aggregates. In an embodiment the invention provides a mixed-mode (MMC) chromatography to produce a composition enriched with fusion protein of interest and substantially reduced pre-peak impurities or aggregates.

In an embodiment, a method for purifying a Fc-fusion protein from a protein sample comprising Fc-fusion protein and Pre-peak impurity:
a) contacting the protein sample onto mixed-mode chromatography column;
b) optionally perform washing the mixed-mode chromatography column;
c) eluting the fusion protein from a mixed-mode chromatography column wherein the eluted fusion protein comprises reduce amount of Pre-peak impurity.

In one aspect of such embodiment, the pre-peak impurity reduced by at least more than about 90%, about 91%, about 92%, about 93%, about 94%, about 95%, about 96%, about 97%, about 98%, about 99%. In an embodiment the pre-peak impurity is below quantifiable limit.

In an embodiment, a method for purifying a Fc-fusion protein from a protein sample comprising:
a) contacting the protein sample onto mixed-mode chromatography column;
b) washing the mixed-mode chromatography column with buffer comprising a suitable additive;
c) eluting the fusion protein from a mixed-mode chromatography column with reduced amount of impurities;
wherein, the suitable additive is arginine or arginine derivatives.

In an embodiment, the concentration of arginine or arginine derivatives is selected from about 20mM to about 100mM.

In another embodiment, the concentration of arginine or arginine derivatives is selected from about 60mM to about 800mM.

In another embodiment, the concentration of arginine or arginine derivatives is selected from about 20mM, about 30mM, about 40mM, about 50mM, about 60mM, about 70mM, about 80mM, about 90mM, about 100mM, about 110mM, about 120mM, about 130mM, about 140mM, about 150mM, about 160mM, about 170mM, about 180mM, about 190mM, about 200mM, about 210mM, about 220mM, about 230mM, about 240mM, about 250mM, about 260mM, about 270mM, about 280mM, about 290mM and about 300mM.

In an embodiment, a method for purifying a Fc-fusion protein from a protein sample comprising:
a) contacting the protein sample onto mixed-mode chromatography column;
b) washing the mixed-mode chromatography column with a suitable buffer comprising suitable additive;
c) eluting the fusion protein from a mixed-mode chromatography column;
wherein the elution buffer comprises arginine or arginine derivatives concentration selected from about below 600mM, preferably 300mM.

In an embodiment, a method for purifying a Fc-fusion protein from a protein sample comprising Fc-fusion protein and impurity:
a) contacting the protein sample onto mixed-mode chromatography column;
b) optionally perform washing the mixed-mode chromatography column;
c) eluting the fusion protein from a mixed-mode chromatography column;
wherein the elution is performed suitable buffer and arginine & the eluted fusion protein comprises reduce amount of impurity.

In an embodiment, the arginine concentration is selected from 0.1M to 0.5M preferably 0.3M.

In such embodiment, the impurities are selected from pre-peak, HMW and LMW.

In an embodiment, the eluted fusion protein from mixed-mode chromatography column comprises reduced amount impurity compared to elution without using arginine or arginine derivatives in wash and elution buffers.

In an embodiment, a process for purification of IgG1 containing protein comprising:
a) collecting the IgG1 containing protein from the suitable mammalian expression system and the impurities;
b) contacting the IgG1 containing protein affinity chromatography;
c) eluting the IgG1 containing protein;
d) contacting the eluted IgG1 containing protein onto mixed-mode chromatography, optionally followed by other suitable purification steps; wherein mixed-mode chromatography is performed in bind-elute mode.

In an embodiment, the invention provides an affinity chromatography and mixed mode chromatography to produce a composition comprising enriched CTLA-4 IgG1 fusion protein and significantly reduced high molecular weight (HMW) impurities selected from about 10%, about 9%, about 8%, about 7%, about 6%, about 5%, about 4%, about 3%, about 2%, about 1%, about 0.5% or less HMW.

In one aspect of this embodiment, the low HMW composition comprises about 0.9% or less HMW, about 0.8% or less HMW, about 0.7% or less HMW, about 0.6% or less HMW, about 0.5% or less HMW, about 0.4% or less HMW, about 0.3% or less HMW, about 0.2% or less HMW, about 0.1% or less HMW.

In an embodiment, the invention provides an affinity chromatography and mixed mode chromatography to produce a composition comprising enriched CTLA-4 IgG1 fusion protein and reduced LMW impurities selected from about 50%, about 40%, about 30%, about 20%, about 10%, about 9%, about 8%, about 7%, about 6%, about 5%, about 4%, about 3%, about 2%, about 1%, about 0.5%, about 0.4%, about 0.3%, about 0.2%, or less LMW or below quantifiable limit.

In an embodiment, a method for purifying a Fc-fusion protein from a protein sample comprising Fc-fusion protein and at least Pre-peak impurity:
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted Fc-fusion protein is substantially free of Pre-peak impurities.

In one aspect of such embodiment, the pre-peak impurity reduced by at least more than 90%, 91%, 92%, 93%, 94%, 95%, 96%, 97%, 98%, 99%. In an embodiment the pre-peak impurity is below quantifiable limit.

In an embodiment, a method for purifying a Fc-fusion protein from a protein sample comprising Fc-fusion protein and at least Low molecular weight (LMW):
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of low molecular weight (LMW) impurities.

In an embodiment, the invention utilizes chromatography column to produce a Fc-fusion protein composition comprising about 0.5% or less LMW, about 0.4% or less LMW, about 0.3% or less LMW, about 0.2% or less LMW, about 0.1% or less LMW.

In an embodiment, a method for purifying a Fc-fusion protein from a protein sample comprising Fc-fusion protein and at least High molecular weight (HMW):
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of high molecular weight (HMW) impurities.

In an embodiment, the Fc-fusion protein composition comprising about 0.5% or less HMW, about 0.4% or less HMW, about 0.3% or less HMW, about 0.2% or less HMW, about 0.1% or less HMW.

In an embodiment, a method for purifying a Fc-fusion protein from a protein sample comprising Fc-fusion protein and undesired glycans:
a) collecting first protein sample/mixture from the suitable mammalian expression system comprising Fc-fusion protein and the impurities;
b) contacting the first protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein, the eluted fusion protein is substantially free of undesired glycans.

In such embodiment, undesired glycans selected from high mannose and afucosylated variants.

In such embodiment, high mannose reduced by less than 80%, preferably less than 77%.

In such embodiment, afucosylated variants are reduced by less than 30%, preferably less than 25%.

In one aspect of such embodiment, the all-column chromatography is performed in bind elute mode.

In one aspect of such embodiment, all column chromatography uses same buffer.

In one aspect of such embodiment, viral inactivation is performed with a suitable detergent for about 60 minutes.

In one aspect of such embodiment, pH-based virus inactivation is not performed in eluate of affinity chromatography.

In an embodiment, the invention is related to the process for the purification of the fusion protein which is free from at least one impurity selected from Host cell proteins (HCP) comprising:
a) collecting the first protein sample/mixture from the suitable mammalian expression system comprising fusion protein and impurities;
b) contacting the protein sample/mixture to affinity chromatography column;
c) eluting the Fc-fusion protein from affinity chromatography column to form second protein mixture;
d) contacting the second protein mixture to mixed-mode chromatography column;
e) eluting the fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
wherein the eluted fusion protein is substantially free of host cell proteins (HCP) impurities.

In an embodiment, the fusion protein comprises reduced HCP impurities at drug substance level after purification selected from about 10 ng/mg or less, about 9 ng/mg or less, about 8 ng/mg or less, about 7 ng/mg or less, about 6 ng/mg or less, about 5 ng/mg or less, about 4 ng/mg or less, about 3 ng/mg or less, about 2 ng/mg or less, about 1 ng/mg or less.

In an embodiment, the invention provides an affinity chromatography and mixed mode chromatography to produce a composition comprising enriched fusion protein and significantly reduced high molecular weight (HMW) impurities about 10%, about 9%, about 8%, about 7%, about 6%, about 5%, about 4%, about 3%, about 2%, about 1%, about 0.5% or less.

In one aspect of this embodiment, the low HMW composition comprises about 0.5% or less HMW, about 0.4% or less HMW, about 0.3% or less HMW, about 0.2% or less HMW, about 0.1% or less.

In an embodiment, the invention provides an affinity chromatography and mixed mode chromatography to produce a composition comprising enriched fusion protein and substantially reduced LMW or fragments.

In such embodiment, LMW reduced by about 10%, about 9%, about 8%, about 7%, about 6%, about 5%, about 4%, about 3%, about 2%, about 1%, about 0.5%, or less.

In an embodiment, the low LMW composition comprises about 0.5% less LMW, about 0.4% or less LMW, about 0.3% or less LMW, about 0.2% or less LMW, about 0.1% or less LMW or below quantifiable limit.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the fusion protein from anion exchange chromatography column to form fourth protein mixture;
h) contacting the fourth protein mixture to hydrophobic interaction chromatography column;
i) eluting the fusion protein from hydrophobic interaction chromatography column to form fifth protein mixture;
   wherein the eluted CTLA-4 IgG1 fusion protein is substantially free of LMW and HMW impurities.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the CTLA-4 IgG1 fusion protein from anion exchange chromatography column to form fourth protein mixture;
h) contacting the fourth protein mixture to hydrophobic interaction chromatography column;
i) eluting the CTLA-4 IgG1 fusion protein from hydrophobic interaction chromatography column to form fifth protein mixture;
   wherein the eluted fusion protein is substantially free of Pre-peak, LMW and HMW impurities.

In an embodiment, the invention is related to the purification of CTLA-4 IgG1 fusion protein from a protein mixture comprising:
a) collecting the first protein sample/mixture from the suitable mammalian expression system comprising fusion protein and impurities;
b) contacting the protein sample/mixture to affinity chromatography column;
c) eluting the CTLA-4 IgG1 fusion protein from affinity chromatography column to form second protein mixture;
d) contacting the second protein mixture to mixed-mode chromatography column;
e) eluting the CTLA-4 IgG1 fusion protein from a mixed-mode chromatography column to form third protein mixture;
f) contacting the third protein mixture to anion exchange chromatography column;
g) eluting the CTLA-4 IgG1 fusion protein from anion exchange chromatography column to form fourth protein mixture;
   wherein the eluted fusion protein is substantially free of Pre-peak, LMW and HMW impurities.

In an embodiment, the invention is related to the process for purification of IgG1 containing protein comprising:
a) obtaining IgG1 protein mixture from the suitable mammalian expression system comprising protein of interest and impurities selected from pre-peak, HMWs, LMWs, and undesired glycan;
b) loading onto Protein A affinity chromatography;
c) eluting from Protein A affinity chromatography;
d) loading the elute onto a Mixed-mode chromatography;
e) eluting from Mixed-mode chromatography;
f) loading the elute onto an anion exchange chromatography;
g) eluting from anion exchange chromatography;
h) loading the elute onto a Hydrophobic interaction chromatography;
i) eluting from a Hydrophobic interaction chromatography;
wherein the eluted protein from Hydrophobic interaction chromatography is optionally followed by other suitable purification steps; wherein the elute comprises substantially higher purity of protein of interest and reduce amount of said impurities.

In an embodiment, the process as reduces more than 95% of the impurity selected from pre-peak, HMWs, and LMWs.

In an embodiment, the process reduces more than 98% of the impurity selected from pre-peak, HMW, and LMW.

In an embodiment, the process wherein the steps (b) to (i) carried out with same buffer at same pH 7.2±0.2.

In an embodiment, the process wherein the other purification method is selected from ultrafiltration and/or diafiltration is performed at least one or two time .

In an embodiment, the invention provides an affinity chromatography, mixed mode chromatography and anion exchange chromatography to produce a composition comprising enriched CTLA-4 IgG1 fusion protein and significantly reduced high molecular weight (HMW) impurities selected from about 10%, about 9%, about 8%, about 7%, about 6%, about 5%, about 4%, about 3%, about 2%, about 1%, about 0.5% or less HMW.

In one aspect of this embodiment, the low HMW composition comprises about 0.9% or less HMW, about 0.8% or less HMW, about 0.7% or less HMW, about 0.6% or less HMW, about 0.5% or less HMW, about 0.4% or less HMW, about 0.3% or less HMW, about 0.2% or less HMW, about 0.1% or less HMW.

In an embodiment, the invention provides an affinity chromatography, mixed mode chromatography and anion exchange chromatography to produce a composition comprising enriched CTLA-4 IgG1 fusion protein and reduced LMW impurities selected from about 50%, about 40%, about 30%, about 20%, about 10%, about 9%, about 8%, about 7%, about 6%, about 5%, about 4%, about 3%, about 2%, about 1%, about 0.5%, about 0.4%, about 0.3%, about 0.2%, or less LMW or below quantifiable limit.

In an embodiment, the invention provides an affinity chromatography, mixed mode chromatography and anion exchange chromatography to produce a composition comprising enriched CTLA-4 IgG1 fusion protein and reduced undesired glycans.

In such embodiment, undesired glycans selected from high mannose and afucosylated variants.

In such embodiment, high mannose reduced by less than 80%, preferably less than 77%.

In such embodiment, afucosylated variants are reduced by less than 30%, preferably less than 25%.

In such embodiment, the high mannose is reduced by about 95%, about 94%, about 93%, about 92%, about 91%, about 90%, about 89%, about 88%, about 87%, about 86%, about 85%, about 84%, about 83%, about 82%, about 81%, about 80%.

In an embodiment, the invention utilizes a chromatography column to produce a Fc-fusion protein composition comprising below about 25% or less acidic variants, about 24% or less acidic variants, about 23% or less acidic variants, about 22% or less acidic variants, about 21% or less acidic variants, about 20% or less acidic variants, about 19% or less acidic variants, about 18% or less acidic variants, about 17% or less acidic variants, about 16% or less acidic variants, about 15% or less acidic variants, about 14% or less acidic variants, about 13% or less acidic variants, about 12% or less acidic variants, about 11% or less acidic variants, about 10% or less acidic variants, about 9% or less acidic variants, about 8% or less acidic variants, about 7% or less acidic variants, about 6% or less acidic variants, about 5% or less acidic variants, about 4% or less acidic variants, about 3% or less acidic variants, about 2% or less acidic variants, about 1% or less acidic variants.

In an embodiment, the invention utilizes a chromatography column to produce a Fc-fusion protein composition comprising below about 25% or less basic variants, about 24% or less basic variants, about 23% or less basic variants, about 22% or less basic variants, about 21% or less basic variants, about 20% or less basic variants, about 19% or less basic variants, about 18% or less basic variants, about 17% or less basic variants, about 16% or less basic variants, about 15% or less basic variants, about 14% or less basic variants, about 13% or less basic variants, about 12% or less basic variants, about 11% or less basic variants, about 10% or less basic variants, about 9% or less basic variants, about 8% or less basic variants, 7% or less basic variants, 6% or less basic variants, 5% or less basic variants, 4% or less basic variants, 3% or less basic variants, 2% or less basic variants, 1% or less basic variants.

In one aspect of such embodiment, the chromatography column selected from mixed-mode chromatography, anion exchange chromatography and hydrophobic interaction chromatography reduces the high molecular weight impurity to amount below selected from about 20 %, about 15 %, about 10 %, about 5 %, or less than about 1 %, about 0.9%, about 0.8%, about 0.7%, about 0.6%, about 0.5%, about 0.4%, about 0.3%, about 0.2%.

In one aspect of such embodiment, the chromatography column selected from mixed-mode chromatography, anion exchange chromatography and hydrophobic interaction chromatography reduces the low molecular weight impurity to amount below selected from about 44 %, about 33 %, about 23 %, about 15 %, or less than about 1.5%, about 1.3%, about 1 %, about 0.9%, about 0.8%, about 0.7%, about 0.6%, about 0.5%, about 0.4%, about 0.3%, about 0.2%.

In an embodiment, the invention provides a mixed mode chromatography and hydrophobic interaction chromatography to produce a composition comprising enriched CTLA-4 IgG1 fusion protein and significantly reduced high molecular weight (HMW) impurities selected from about 10%, about 9%, about 8%, about 7%, about 6%, about 5%, about 4%, about 3%, about 2%, about 1%, about 0.5%, about 0.4%, about 0.3%, about 0.2% or less HMW.

In one aspect of this embodiment, the low HMW composition comprises about 0.9% or less HMW, about 0.8% or less HMW, about 0.7% or less HMW, about 0.6% or less HMW, about 0.5% or less HMW, about 0.4% or less HMW, about 0.3% or less HMW, about 0.2% or less HMW, about 0.1% or less HMW.

In an embodiment, the invention provides a mixed mode chromatography and hydrophobic interaction chromatography to produce a composition comprising enriched CTLA-4 IgG1 fusion protein and reduced LMW impurities selected from about 50%, about 40%, about 30%, about 20%, about 10%, about 9%, about 8%, about 7%, about 6%, about 5%, about 4%, about 3%, about 2%, about 1%, about 0.5%, about 0.4%, about 0.3%, about 0.2% or less LMW or below quantifiable limit.

In an embodiment, the affinity chromatography column resin is selected from Protein A resin, Protein G resin, preferably Protein A resin. Protein A column chromatography resin is selected from Toyopearl AF-rProtein A HC-650, Mab Select Sure LX, MabSelect SuRe, MabSelectXtra, ProSep Ultra Plus, and Eshmuno A.

In an embodiment, the first step of affinity chromatography comprises clarified harvest cell culture fluid (HCCF) that is obtained from suitable mammalian expression system. The pH of HCCF is adjusted to pH selected from about pH 8 to about pH 9, preferably pH 7.2±0.2 with 2 M Tris base just before loading onto the affinity column.

In another embodiment, the clarified harvest cell culture fluid (HCCF) can be loaded directly on to the affinity chromatography without any pH adjustment prior.

In an embodiment, the pH of the clarified harvest cell culture fluid (HCCF) is from about 7.2±0.2.

In an embodiment, prior to loading the Protein A column is equilibrated with a suitable buffer. In an embodiment, the suitable buffer is selected from Tris Acetate, Tris-HCl buffer, Sodium Phosphate (NaPo4), Sodium Chloride (NaCl), HEPES, Triethanolamine, Borate, Glycine-NaOH.

In another aspect of embodiment, the concentration of equilibration buffer used in affinity chromatography is selected from about 10 mM to about 200 mM.

In preferred embodiment, the concentration of buffer is Tris-HCl or Sodium Phosphate (NaP) is selected from about 5mM to about 30mM, preferably 20mM. In another embodiment, the Tris-HCl or Sodium Phosphate (NaP) is used with Sodium chloride (NaCl) selected from about 50mM to about 200mM. In another embodiment, the Tris-HCl or Sodium Phosphate (NaP) is used with Sodium chloride (NaCl) selected from about 120mM to about 170mM at pH ranging from about 6.8 to about 7.5 and conductivity is selected from about from 10 mS/cm to about 25 mS/cm, preferably about 13 mS/cm.

In an embodiment, the present invention provides a purification process using the same buffer in more than one chromatography column or in all chromatography columns which makes the process more economic at large scale.

In an embodiment, the buffer is sodium phosphate is used in all the chromatography column.

In an embodiment, the concentration of buffer is 20mM Sodium Phosphate (NaP) or Tris HCl and 150 mM Sodium chloride (NaCl), pH about 7.2±0.2 used to equilibrate the affinity column with at least one column volumes, preferably for four column volume. In another embodiment, the concentration of buffer is 20mM Sodium Phosphate (NaP) and 100 mM Sodium chloride (NaCl), pH about 7.2±0.2 used to equilibrate the affinity column with at least one column volumes, preferably for five column volume. The flow rate can be selected from at about 50 cm/hr to at about 300 cm/hr, preferably 300 cm/hr.

In an embodiment, the loading of protein on column, the Protein A column can be washed one or more times by using the equilibrating buffer or by employing different buffers. The Protein A column is first washed with the equilibration buffer for at least 4 to 6 column volumes. This wash can optionally be followed by one or more wash. In another embodiment, the wash buffer is selected from urea, tween 80, isopropanol, Sodium Phosphate (NaP), Sodium chloride (NaCl), EDTA, Tris acetate, Tris-HCl, Sodium chloride (NaCl), HEPES, Triethanolamine, Borate and Glycine-NaOH.

In an embodiment, the concentration of wash buffer selected from about 5 mM to about 100 mM. In another embodiment, the concentration of wash buffer selected from about 10 mM to about 400 mM. In another aspect of embodiment, the concentration of wash buffer selected from about 10 mM to about 200 mM.

In an embodiment, the concentration of wash buffers is selected from 5 mM to about 200 mM and the pH of wash buffer is ranging from pH about 2.0 to about 7.5.

In an embodiment, Protein A column comprises three wash buffers.
a) first wash buffer comprises Tris HCl or Sodium Phosphate (NaP) concentration selected from about 5mM to about 30mM and Sodium chloride (NaCl) concentration selected from about 10mM to about 150mM preferably100mM Sodium chloride (NaCl), at pH 7.0±0.2 or pH 7.2±0.2, conductivity 12.5±2 mS/cm.
b) second wash buffer comprises Sodium Phosphate (NaP) or Tris HCl concentration selected from about 5mM to about 20mM and Sodium chloride (NaCl) concentration from about 1M to about 10M at pH 7.0±0.2 or pH 7.2±0.2, conductivity 87±7 mS/cm.
c) third wash buffer comprises Sodium Phosphate (NaP) concentration selected from 5mM to about 20mM at pH 7.0±0.2 or pH 7.2±0.2, conductivity 2.7±0.3 mS/cm.

In an embodiment, the first wash buffer comprises 20mM Sodium Phosphate (NaP) and 100 mM Sodium chloride (NaCl) at pH 7.2±0.2.

In an embodiment, the second wash buffer comprises 20 mM Sodium Phosphate (NaP) and 1M Sodium chloride (NaCl) at pH 7.2±0.2.

In an embodiment, the third wash buffer comprises 20mM Sodium Phosphate (NaP) at pH 7.2±0.2.

In an embodiment, the loaded protein mixture in Protein A column can then be eluted using an appropriate suitable buffer. The pH of elution buffer is selected from about 2.0 to 3.9, preferably about 3.5±0.2.

In an embodiment, the concentration of elution buffer selected from about 5 mM to about 200 mM. In another embodiment, the concentration of elution buffer selected from about 25 mM to about 150 mM. In another aspect of embodiment, the concentration of elution buffer selected from about 100 mM to about 120 mM.

In an embodiment, the elution buffer comprises Tris or Acetate concentration selected from about 100mM to about 200 mM Tris or Acetate, preferably about 100mM to about 110 mM Tris or Acetate at pH 3.5±0.2, the conductivity is selected from about 0.2 to about 1.0 mS/cm, preferably about 0.5±0.3 mS/cm.

In an embodiment, the concentration of neutralization wash buffer selected from Tris HCl, Sodium Phosphate, Sodium Chloride, and the concentration of neutralization wash buffer selected from about 5 mM to about 200 mM.

In an embodiment, the Protein A column further comprises neutralization wash with 20mM Tris HCl at pH 7.2±0.2.

In an embodiment, the Protein A column further comprises neutralization wash with 20mM Sodium Phosphate (NaP) and 100mM Sodium chloride (NaCl) at pH 7.2±0.2.

In an embodiment, the eluted fractions are collected from about ascending 0.10AU/cm to descending about 0.30AU/cm.

In an embodiment, the eluted fractions are collected from ascending 0.25AU/cm to descending about 0.25AU/cm.

In an embodiment, the second protein mixture obtained from affinity chromatography column is subjected to suitable treatment to make the protein mixture suitable for loading onto MMC.

In another embodiment, the second protein mixture obtained from affinity chromatography column is subjected to neutralization, adjusted pH 7.2±0.2, conductivity preferably 4.0 mS/cm with 1 M tris base.

In an embodiment, the second protein mixture obtained from affinity chromatography column after neutralization is subjected to a mixed mode chromatography column and the eluate obtained from MMC column referred as third protein mixture which has reduced level of at least one impurity selected from Host cell proteins (HCP), Host cell DNA (HCD), Pre-peak, Low molecular weight (LMW) and High molecular weight (HMW).

Mixed-mode chromatography column can be selected from Nuvia aPrime 4A, Capto adhere ImpRes, Capto adhere (N-Benzyl-N-methyl ethanol amine as ligand), Capto MMC (MMC ligand), MEP Hypercel (4-marcaptomethyl pyridine as ligand), HEA Hypercel (hexyl amine as ligand), PPA Hypercel (phenylpropylamine as ligand) exhibit many functionalities for interaction. These resins exhibit multiple functionalities for interaction. The most pronounced are ionic interaction, hydrogen bonding and hydrophobic interaction.

In an embodiment, the mixed-mode chromatography is performed in the bind and elute mode. In another embodiment, the mixed-mode chromatography is performed in flow through mode. In an embodiment, the loading of the protein mixture from Protein A chromatography column to mixed-mode chromatography column, the conductivity of the sample is from about 3.0 mS/cm to about 4.2 mS/cm, preferably 4.0 ms/cm.

In an embodiment, the mixed mode column is equilibrated with suitable buffer selected from histidine hydrochloride, Tris acetate, Tris-HCl sodium citrate, Sodium phosphate (NaP), Arginine HCL, citrate at pH selected from about pH 6 to about pH 9, preferably pH 7.2±0.2, conductivity is selected from about 2.0 mS/cm to about 3.5 mS/cm, preferably 2.7±0.3ms/cm.

In another embodiment, the concentration of equilibration buffer selected from about 10 mM to about 100 mM. In another aspect of one embodiment, the concentration of equilibration buffer selected from about 10 mM to about 80 mM.

In an embodiment, the equilibration buffer comprises Sodium Phosphate (NaP) at pH 7.2±0.2. In an embodiment, the equilibration buffer comprises 20mM Sodium Phosphate (NaP) at pH 7.2±0.2.

In a preferred embodiment, the protein mixture is loaded onto the MMC column. In another embodiment the flow rate can be selected from at about 50 cm/hr to at about 300 cm/hr, preferably 250 cm/hr.

In an embodiment, the wash buffer selected from Tris acetate, Tris-HCl sodium citrate, Sodium phosphate (NaP), Arginine HCl at pH about 6 to about pH 9 preferably pH about 7 to about 7.2. In an embodiment, MMC comprises two wash buffers.

In another embodiment, the concentration of wash buffer selected from about 10 mM to about 100 mM. In another embodiment, the concentration of wash buffer selected from about 10 mM to about 100 mM. In certain embodiment, the wash buffer comprises Arginine or salt thereof selected from more than 50mM. In certain embodiment, the wash buffer comprises Arginine or salt thereof selected from more than 50mM to 100mM, preferably 60mM.

In an embodiment, first wash buffer comprises 20mM Sodium Phosphate (NaP), at pH 7.2±0.2.

In an embodiment, second wash buffer comprises 20mM Sodium Phosphate (NaP), and 60mM Arginine HCl at pH 7.2±0.2.

In an embodiment, second wash at the same pH as of the first wash buffer and/or a conductivity higher than the first wash buffer.

In an embodiment, second wash comprises conductivity selected from about 5 mS/cm to about 25 mS/cm, preferably 7.2±0.5 mS/cm.

In an embodiment, the elution buffer comprises selected from tris acetate, sodium citrate, Sodium phosphate (NaP) and suitable additive selected from arginine and histidine, at pH about 6.5 to about 7.5.

In an embodiment, the elution buffer comprises Tris Hcl, Sodium phosphate concentration selected from about 10 mM to about 100 mM. In another aspect of embodiment, the concentration of elution buffer selected from about 50 mM to about 100 mM.

In an embodiment, the elution buffer comprises Sodium Phosphate (NaP) concentration selected from 10 mM to about 100 mM. In an embodiment the elution buffer comprises Sodium Phosphate (NaP) concentration selected from about 10mM, about 20mM, about 30mM, about 40mM, about 50mM, about 60mM, about 70mM, about 80mM, about 90mM and about 100mM.

In another embodiment, the elution buffer comprises Arginine HCl concentration selected from about 0.1 M to about 1 M. In an embodiment, the elution buffer comprises Arginine HCl concentration selected from about 0.1M, about 0.2M, about 0.3M, about 0.4M, about 0.5M, about 0.6M, about 0.7M, about 0.8M, about 0.9M, and about 1 M.

In an embodiment, the elution buffer comprises Sodium Phosphate (NaP) and Arginine HCl at pH about pH 7.2±0.2. In an embodiment, the elution buffer comprises 20mM Sodium Phosphate (NaP) and 0.3 M Arginine-HCl at pH 7.2±0.2.

In an embodiment, the elution is performed with an appropriate buffer. In preferred embodiment, the elution buffer can be one or mixture of more than one buffer. In an embodiment, the protein mixture is eluted by using Sodium Phosphate (NaP) concentration selected from about 1 mM to about 20 mM Sodium Phosphate (NaP) and Arginine HCl concentration selected from about 0.1M to about 1M at pH 7.2±0.2, conductivity is selected from about 5 mS/cm to about 25 mS/cm, preferably 21±3 mS/cm.

In an embodiment, the gradient of elution is performed in linear gradient wherein the concentration of elution buffer increased from 0 to about 80% preferably 0 to about 30%, more preferably about 20% to about 80%. In an embodiment, the gradient was performed for column volume selected from about 1CV, about 2CV, about 3CV, about 4CV, about 5CV, about 6CV, about 7CV, about 8CV, about 9CV, about 10CV, about 11CV, about 12CV, about 13CV, about 14CV, about 15CV, about 16CV, about 17CV, about 18CV, about 19CV, and about 20CV. In preferred embodiment, the gradient was performed for column volume about 10 CV.

In an embodiment, the eluted fractions are collected from about ascending 0.10AU/cm to about descending 4.0AU/cm.

In preferred embodiment, the eluted fractions are collected from about ascending 0.1AU/cm to about descending 3.0AU/cm.

In an embodiment, the eluted fractions are collected from about ascending 0.5AU/cm to about descending 1.5AU/cm.

In an embodiment, the protein mixture obtained from mixed mode chromatography column is subjected to viral inactivation or removal, optionally further comprises one or more chromatography steps.

In one aspect of such embodiment, the protein mixture obtained from mixed mode chromatography column is incubated with detergent or surfactant for viral inactivation selected from about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 70 minutes, about 80 minutes.

In one aspect of such embodiment, the detergent or surfactant is selected from Triton X100 or Octo X100, sugar-based detergent *n*-dodecyl-β- D-maltoside, preferably Triton X100.

In an embodiment, the viral inactivated protein mixture subjected to filtration before loading onto anion exchange chromatography.

In one aspect of such embodiment, the filtrate of viral inactivated protein mixture is further subjected for anion exchange load preparation before loading on to anion exchange chromatography column. In another embodiment, the protein mixture obtained from MMC column subjected to an anion exchange chromatography column and the eluate obtained from anion exchange column can be referred as fourth protein mixture which have reduced level of impurities selected from Pre-peak, HMW and LMW.

In one embodiment, the anion exchange chromatography is selected from Poros XQ, Poros HQ DEAE, Sepharose fast flow, Fractogel^{®} EMD DEAE (M), Toyopearl DEAE-650, Toyopearl DEAE-650, Nuvia Q.

In an embodiment, the anion exchange is strong anion exchange preferably Poros XQ.

In another embodiment, the present invention uses anion exchange chromatography (AEX) column removes impurities selected from Host cell proteins (HCP), Host cell DNA (HCD), Prepeak, Low molecular weight (LMW) glycan variants and High molecular weight (HMW).

In another embodiment, the anion exchange chromatography reduces undesired glycan selected from high mannose by less than 50%, preferably less than 77% and afucosylated glycans by less than 50%, preferably less than 25%.

In another embodiment, the anion exchange is performed in bind elute mode comprising: (i) loading the eluted IgG1 protein at suitable pH and/or conductivity for binding (ii) performing washing at suitable pH and/or conductivity (iii) eluting the IgG1 protein at suitable pH and/or conductivity.

In another embodiment, the loading is performed at suitable pH selected from about 6 to about 8 and conductivity selected from about 5 mS/cm to about 8 mS/cm.

In another embodiment, the suitable pH and/or conductivity is maintained with suitable buffer selected from sodium phosphate, Tris-HCl, sodium chloride.

In another embodiment, the suitable washing in AEX comprises: (i) first wash with equilibration buffer at suitable pH 7.2±0.2 and/or conductivity 2.7±0.3 mS/cm;
(ii) second wash at the same pH as of the first wash buffer and/or a conductivity higher than the first wash buffer selected from about 5mS/cm to about 20mS/cm.

In another embodiment, the second wash buffer is selected from sodium phosphate and sodium chloride.

In an embodiment, the anion exchange chromatography is performed in the bind and elute mode. In another embodiment, the anion exchange chromatography is optionally performed in flow through mode. In an embodiment, the loading of the protein mixture from MMC column to anion exchange chromatography column.

In an embodiment, pH of the anion exchange load is 7.2±0.2, conductivity of the sample is adjusted by diluting with WFI ratio selected from about 1:1, 1:1.1, 1:1.2, 1:1.3, 1:1.4, 1:1.5, more preferably 1:1.3.

In another embodiment, the conductivity of anion exchange load is adjusted with WFI from about 6.5 mS/cm.

In an embodiment, the anion exchange chromatography column is equilibrated with suitable buffer selected from histidine hydrochloride, tris acetate, sodium citrate, Sodium phosphate (NaP), citrate, Sodium chloride (NaCl) preferably Sodium phosphate (NaP) and Sodium chloride (NaCl) at pH selected from about 6 to about pH 9, preferably at pH 7.2±0.2, conductivity is selected from about 2.0 mS/cm to about 3.2 mS/cm, preferably 2.7±0.3 ms/cm.

In an embodiment, the concentration of equilibration buffer selected from about 5 mM to about 50 mM. In another embodiment, the concentration of selected from about 10 mM to about 50 mM. In another aspect of embodiment, the concentration is selected from about 15 mM to about 30 mM.

In an embodiment, the equilibration buffer comprises Sodium phosphate (NaP) at pH 7.5. In an embodiment, the equilibration buffer comprises 20mM Sodium phosphate (NaP) at pH 7.2±0.2.

In an embodiment, the load preparation done with MMC OP diluted with WFI in ratio 1:1.33 at pH 7.2±0.2. In a preferred embodiment, the protein mixture is loaded onto the AEX column. In another embodiment the flow rate can be selected from at about 50 cm/hr to at about 500 cm/hr, preferably 300 cm/hr.

In an embodiment, the wash buffer selected from Tris acetate, Tris, Sodium citrate, Sodium chloride (NaCl), Sodium phosphate (NaP), at pH about 6.0 to about 9.0.

In an embodiment, the concentration of wash buffer selected from about 5 mM to about 30 mM. In another embodiment, the concentration of wash buffer selected from about 10 mM to about 30 mM. In another aspect of embodiment, the concentration of wash buffer selected from about 10 mM to about 20 mM.

In an embodiment, the first wash buffer comprises about 20mM to 30mM Sodium phosphate (NaP) at pH 7.2±0.2.

In an embodiment, the second wash buffer comprises about 15mM to about 30mM Sodium phosphate (NaP) and less than 80mM Sodium chloride (NaCl) preferably less than 60mM NaCl at pH 7.2±0.2, and conductivity is selected from about 2.0 mS/cm to about 3.2 mS/cm, preferably 2.7±0.3 ms/cm. In an embodiment, the second wash performed using step gradient selected from about 10%, 15%, 20%, 25%, 30% of buffer B, preferably 20% of buffer B in 5 CV.

In an embodiment, the elution buffer selected from Tris acetate, Acetate, Sodium citrate, Sodium chloride (NaCl), Sodium phosphate (NaP), at pH about 6.5 to about 7.5.

In an embodiment, the concentration of the high salt buffer used in elution buffer selected from about more than about 100 mM. In an embodiment, the concentration of high salt buffer is selected from about 100mM to about 1050mM. In an embodiment, the concentration of high salt buffer is selected from about 200mM to about 325mM.

In an embodiment, the elution buffer comprises Sodium phosphate (NaP) concentration selected from about10mM to about 50mM. In an embodiment, the elution buffer comprises Sodium phosphate (NaP) concentrated selected from about 10mM, about 20mM, about 30mM, about 40mM, about 50mM, about 60mM, about 70mM, about 80mM, about 90mM and about 100mM.

In another embodiment, the elution buffer comprises Sodium chloride (NaCl) concentration selected from about 0.1 M to about 1 M. In an embodiment, the elution buffer comprises Sodium chloride (NaCl) concentration selected from about 0.1mM, about 0.2mM, about 0.3mM, about 0.4mM, about 0.5mM, about 0.6mM, about 0.7mM, about 0.8mM, about 0.9mM, about 1mM, about 1.2M and about 1.5M.

In an embodiment, the elution is performed with an appropriate buffer. In another embodiment, the elution buffer can be one or mixture of more than one buffer.

In an embodiment, the protein is eluted by elution buffer comprising about 10mM to about 50mM Sodium phosphate (NaP) and about 0.1 to about 1 M Sodium chloride (NaCl), preferably 20mM Sodium phosphate (NaP) and 0.3M Sodium chloride (NaCl) at pH 7.2±0.2, and conductivity is selected from about 20 mS/cm to about 50 mS/cm, preferably 30±3 mS/cm.

In an embodiment, the gradient was performed for column volume selected from about 1CV, about 2CV, about 3CV, about 4CV, about 5CV, about 6CV, about 7CV, about 8CV, about 9CV, about 10CV, about 11CV, about 12CV, about 13CV, about 14CV, about 15CV, about 16CV, about 17CV, about 18CV, about 19CV and about 20CV.

In an embodiment, the gradient of elution buffer is performed in anion exchange chromatography column for elution from about 20% to about 80% of buffer B, preferably about 20% to about 70% of about 10 CV to about 20CV, preferably 15CV. In certain embodiment, the elution is performed using step and/or linear gradient selected from about 1% to about 10% step gradient and 10%, 15%, 20%, 25%, 30% of linear gradient of buffer B, preferably 20% of buffer B in about 15 CV to about 25 CV, more preferably in 15CV.

In an embodiment, the eluted fractions are collected from about ascending 10mAU/cm to about descending 80mAU/cm in a fixed CV. In an embodiment, the eluted fractions collected from ascending of about 10mAU/cm to descending of about 100mAU/cm.

The anion exchange chromatography reduces undesired glycan selected from high mannose & afucosylation. In certain embodiment, the anion exchange chromatography reduces undesired glycan selected from high mannose & afucosylation by less than 50% preferably less than 70%.

In another embodiment, the use of anion exchange chromatography (AEX) process for the purification of fusion protein from mixture comprising protein of interest and High molecular weight (HMW), Low molecular weight (LMW) impurities.

In another embodiment, the protein mixture obtained from AEX column subjected to a hydrophobic interaction chromatography column and the eluate obtained from anion exchange column can be referred as fourth protein mixture which have reduced level of impurities selected from Pre-peak, HMW and LMW.

In another embodiment, the anion exchange chromatography is strong anion chromatography.

In another embodiment, the present invention includes only one strong anion exchange chromatography.

In another embodiment, the present invention does not include more than one strong anion exchange chromatography.

In an embodiment, the hydrophobic interaction chromatography resin is selected from Poros Benzyl, Butyl Toyopearl 650 M resin, Toyopearl Phenyl-650, Butyl Sepharose 6 Fast Flow, Phenyl Sepharose 6 Fast Flow, Butyl Sepharose HP, Phenyl Sepharose 6 Fast Flow high sub, Capto Phenyl high sub, Capto Butyl impRes.

In embodiment, the hydrophobic interaction chromatography is optionally performed in flow through mode. In another embodiment, the hydrophobic interaction chromatography is performed in bind-elute mode. In an embodiment, the AEX OP or fourth protein mixture is mixed with suitable buffer at 1:1 ratio prior to loading on to the HIC column, the final conductivity reaches to about from 40 mS/cm to about 70 mS/cm, preferably about 48 mS/cm to 52 mS/cm. In an embodiment, suitable buffer is selected from at least one or any combination of the salts selected from Tris acetate, Sodium phosphate (NaP), Sodium citrate, disodium hydrogen phosphate anhydrous, Trisodium citrate dihydrate, Histidine-HCl, Imidazole, bis-tris, maleate, preferably Sodium phosphate (NaP) and sodium citrate at pH selected from about pH 6 to about 9, preferably pH 7.20±0.2 and conductivity is selected from about from 50 mS/cm to about 80 mS/cm, preferably about 62.0±5.0 mS/cm.

In an embodiment, the buffers are selected from Tris acetate, Sodium citrate, Sodium phosphate (NaP), disodium hydrogen phosphate anhydrous, Trisodium citrate dihydrate, Histidine-HCl, Imidazole, bis-tris, and maleate.

In an embodiment, the concentration of buffer comprises Sodium Phosphate (NaP) concentration selected from about 10mM to about 100mM. In an embodiment, the concentration of high salt buffer comprises Sodium phosphate (NaP) concentration selected from about 15mM, about 20mM, about 25mM, about 30mM, about 35mM, about 40mM, about 45mM, about 50mM, about 55mM, about 60mM, about 65mM, about 70mM, about 75mM, about 80mM, about 85mM, about 90mM, about 95mM and about 100mM.

In an embodiment, the concentration of Sodium citrate concentration selected from about 0.1 M to about 1.0 M. In an embodiment, the concentration of high salt buffer comprises Sodium citrate concentration selected from selected from about 0.1M, about 0.2M, about 0.3M, about 0.4M, about 0.5M, about 0.6M, about 0.7M, about 0.8M, about 0.9M, about 1M, about 1.2M and about 1.5M.

In an embodiment, the equilibration buffer comprises Sodium phosphate (NaP) and Sodium citrate at pH from about 6 to about 8. In an embodiment, the equilibration buffer comprises 20mM Sodium phosphate (NaP) and 0.4M Sodium citrate at pH 7.2±0.2.

In an embodiment, the equilibration buffer conductivity is selected from about 35 mS/cm, about 40 mS/cm, about 45 mS/cm, about 50 mS/cm, preferably about 45.0±4.0 mS/cm.

In an embodiment, the wash buffer selected from tris acetate, tris, sodium citrate, Sodium chloride (NaCl), Sodium phosphate (NaP), at pH about 6 to about 9.

In an embodiment, the concentration of sodium phosphate used in wash buffer selected from about 10 mM to about 50 mM. In another embodiment, the concentration of wash buffer selected from about 10 mM to about 30 mM. In another aspect of embodiment, the concentration of wash buffer selected from about 10 mM to about 20 mM.

In an embodiment, the concentration of sodium citrate used in wash buffer selected from about 0.1 M to about 0.5 mM.

In an embodiment, the wash comprises 20mM sodium phosphate and 0.4M sodium citrate at pH 7.2±0.2.

In an embodiment, the HIC wash buffer conductivity is selected from about 35 mS/cm, about 40 mS/cm, about 45 mS/cm, about 50 mS/cm, preferably about 45.0±4.0 mS/cm.

In an embodiment, the HIC load preparation done at 1:1 dilution of AEX OP with HIC stock buffer.

In an embodiment, the HIC stock buffer is about 20mM to about 50mM preferably 40mM Sodium phosphate (NaP) and 0.6M to about 1M sodium citrate preferably 0.8M sodium citrate, pH adjusted to 7.2±0.2 before loading and conductivity is selected from about 30 mS/cm to about 70 mS/cm, preferably 50±0.2 mS/cm.

In an embodiment, the hydrophobic interaction chromatography is performed in bind-elute mode.

In an embodiment, the hydrophobic interaction chromatography reduces impurity selected from Prepeak, HMWs,andLMWs by less than 50%, preferably less than 70%.

In an embodiment, the bind-elute mode comprises (i) loading the eluted IgG1 protein at suitable pH and/or conductivity for binding (ii) performing washing at suitable pH and/or conductivity (iii) eluting the IgG1 protein at suitable pH and/or conductivity.

In an embodiment, the suitable washing is performed with buffer selected from sodium phosphate, sodium citrate at pH 7.2±0.2 and conductivity selected from about 35 mS/cm to about 50 mS/cm, preferably about 45.0±4.0 mS/cm.

In an embodiment, the elution is performed with suitable buffer selected from sodium phosphate, sodium citrate at suitable pH 7.2±0.2 and conductivity 2.7±0.3 mS/cm.

In an embodiment, the elution buffer selected from tris acetate, acetate, sodium citrate, Sodium chloride (NaCl), Sodium phosphate (NaP), at pH about 6 to about 9 preferably 7.2±0.2.

In an embodiment, the concentration of elution buffer selected from about 0.1 mM to about 50mM. In another aspect of embodiment, the concentration of elution buffer is selected from about 0.1 mM to about 30 mM.

In an embodiment, the elution is performed in linear gradient. In an embodiment, the elution buffer comprises Sodium phosphate (NaP) concentration selected from 0.1 mM to about 40 mM. In an embodiment, the elution buffer comprises Sodium phosphate (NaP) concentration selected from about 0.1mM, about 0.2mM, about 0.3mM, about 0.4mM, about 0 .5mM, about 0.6mM, about 0.7mM, about 0.8mM, about 0.9mM, about 1mM, about 2mM, about 3mM, about 4mM, about 5mM, about 6mM, about 7mM, about 8mM, about 9mM, about 10mM, about 11mM, about 12mM, about 13mM, about 14mM, about 15mM, about 16mM, about 17mM, about 18mM, about 19mM, about 20mM, about 25mM, about 30mM, about 35mM, and 40mM. In an embodiment, the elution buffer comprises Sodium phosphate (NaP) at pH 7.2±0.2, and conductivity is selected from about 2 mS/cm to about 5 mS/cm, preferably 2.7±0.3 mS/cm. In an embodiment the elution buffer comprises 20mM Sodium phosphate (NaP). In another embodiment, the flow rate can be selected from at about 50 cm/hr to at about 400 cm/hr, preferably 300 cm/hr.

In certain embodiment, the gradient was performed for column volume selected from about 1CV, about 2CV, about 3CV, about 4CV, about 5CV, about 6CV, about 7CV, about 8CV, about 9CV, about 10CV, about 11CV, about 12CV, about 13CV, about 14CV, about 15CV, about 16CV, about 17CV, about 18CV, about 19CV and about 20CV.

In an embodiment, elution from HIC is carried out by performing linear gradient from 0% of elution buffer B to about 100% of elution buffer B linear gradient in about 10 to about 25 CV, preferably 15 column volumes. In an embodiment, the eluted fractions are collected from ascending about 10mAU/cm to descending about 300mAU/cm.

In an embodiment, the eluted fractions are collected from ascending about 1.5AU/cm to descending about 2.0AU/cm. In an embodiment, the eluted fractions are collected from ascending about 1.85AU/cm to descending about 2.0AU/cm.

In an embodiment, the process further comprises regeneration buffer. In an embodiment the regeneration buffer is WFI.

In another embodiment, the use of hydrophobic interaction chromatography (HIC) process for the purification of fusion protein from mixture comprising protein of interest and high molecular weight (HMW) impurities.

In an embodiment, the third protein mixture obtained from anion exchange chromatography subjected to a hydrophobic interaction chromatography column and the eluate obtained from hydrophobic interaction chromatography column called as fourth protein mixture which has reduced level impurities selected from HMW and LMW.

In an embodiment, the protein is collected in fractions in the bind and elute (BE) mode. In an embodiment, the elute fractions contains protein of interest and substantially free from high molecular weight (HMW) and other impurities.

In an embodiment, of the invention is related to the purification of fusion protein from the protein mixture by using mixed-mode chromatography column that can be performed at any step after affinity chromatography column.

In an embodiment, the reduction in pre-peak impurity selected from about 25%, about 24%, about 23%, about 22%, about 21%, about 20%, about 19 %, about 18%, about 17%, about 16%, about 15%, about 14%, about 13%, about 12%, about 11%, about 10%, about 5%, about 3%, about 1%, or below quantifiable limit.

In an embodiment, the MMC column reduces impurities more than about 95% or more, about 96% or more, about 97% or more, about 98% or more and about 99% or more.

In an embodiment, the elute fractions contain substantially purified protein of interest and reduced Host cell proteins (HCP), Host cell DNA (HCD), Low molecular weight (LMW) and High molecular weight (HMW) impurities.

In an embodiment, the elute is collected and can be referred as final protein of interest.

In an embodiment, the protein mixture obtained from HIC column is further subjected to ultrafiltration and diafiltration with reduced level of impurities.

In an embodiment, the protein mixture obtained from HIC column is subjected to virus filtration before ultrafiltration and diafiltration.

In another embodiment, the viral filtration is performed with pore size with 20nm (Planova 20N).

In one aspect of such embodiment, the filtrates are substantially free from viruses, adventitious agents, LMW, HMW and pre-peak.

In an embodiment, the pharmaceutical composition of CTLA4-IgG1 or CTLA4-Fc fusion protein comprises substantially purified monomer of said fusion protein having purity at least 90% and HMW less than about 0.2%, measure by SE-HPLC.

In an embodiment, the pharmaceutical composition of CTLA4-IgG1 or CTLA4-Fc fusion protein comprises substantially purified monomer of said fusion protein having purity at least 90% and LMW less than about 0.3%, measure by SE-HPLC.

In an embodiment, the pharmaceutical composition of CTLA4-IgG1 or CTLA4-Fc fusion protein comprises substantially purified monomer of said fusion protein having purity at least about 90% and pre-peak is less than about 0.2%, measure by SE-HPLC.

In an embodiment, the pharmaceutical composition of CTLA4-IgG1 or CTLA4-Fc fusion protein monomer purity is selected from more than about 90% or about 91% or about 92% or about 93% or about 94% or about 95% or about 96% or about 97% or about 98% or about 99%.

In an embodiment, the fusion protein comprises reduced HCP impurities at drug substance level after purification selected from about 10 ng/mg or less, about 9 ng/mg or less, about 8 ng/mg or less, about 7 ng/mg or less, about 6 ng/mg or less, about 5 ng/mg or less, about 4 ng/mg or less, about 3 ng/mg or less, about 2 ng/mg or less, about 1 ng/mg or less.

The present invention provides a following example for illustration purpose and the scope of the present invention should not be considered limiting to the present examples.

### Example 1 - Purification of Fc-fusion protein by Affinity Chromatography followed by MMC (Mix mode Chromatography).

All chromatographic processes were carried out using an AKTA Pure 150 system from Cytiva (formally known as GE Healthcare). Concentration of protein samples were determined by measuring absorbance at 280nm using Shimadzu Spectrophotometer. Protein A Chromatography resin (Mab Select Sure LX) obtained from Cytiva and MMC resin (Nuvia aPrime 4A) obtained from Bio-rad.

A Fc-fusion protein expressed in Chinese Hamster Ovary (CHO) cell line is captured using Protein A resin (Mab Select Sure LX, Cytiva) packed in XK 50/30 column. Eluted protein is further purified using Mix Mode chromatography resin (Nuvia aPrime 4A, BioRad) packed in XK 50/30.

Fc-Fusion protein is obtained from the suitable mammalian expression system comprising Fc-fusion protein which is CTLA4-IgG1. Fc-fusion protein present in the sample to a Protein A chromatography resin and eluting the Fc-fusion protein from the Protein A resin for which experiment design is shown in table 1, wherein the eluted product was neutralized and referred to as Neutralized Protein A Elute (NPEL). The residence time is 4 min for all the phases.

Binding the NPEL to a mixed mode chromatography resin (MMC) and eluting the sample from the MMC resin by increasing arginine concentration for which experiment design is shown in table 2, referred to as Mix mode chromatography output (MMC OP), the residence time is 5 min for all the phases.

**Table 1. Experiment Design for Affinity chromatography**

| **Step** | **Buffer** | **Residence Time (min)** | **Column Volume (CV)** |
|---|---|---|---|
| Storage buffer removal | WFI | 4 | 3 |
| Sanitization | 0.1 M NaOH | 4 | 3 |
| Equilibration | 20mM Sodium Phosphate (NaP) + 100 mM Sodium chloride (NaCl), pH 7.2 ± 0.2 | 4 | 5 |
| Load | Clarified Harvest | 4 | Till loading volume |
| Wash 1 | 20mM Sodium Phosphate (NaP) +100mM Sodium chloride (NaCl), pH 7.2 ± 0.2 | 4 | 3 |
| Wash 2 | 20mM Sodium Phosphate (NaP) + 1M Sodium chloride (NaCl), pH 7.2 ± 0.2 | 4 | 3 |
| Wash 3 | 20 mM Sodium Phosphate (NaP), pH 7.2 ± 0.2 | 4 | 5 |
| Elution | 110 mM Acetate, pH 3.5 ± 0.1 | 4 | 5 |
| Neutralization wash | 20mM Sodium Phosphate (NaP) + 100 mM Sodium chloride (NaCl), pH 7.2 ± 0.2 | 4 | 3 |
| Sanitization | 0.1 N NaOH | 4 | 3 |
| Storage | 2% Benzyl Alcohol in Wash 3 buffer | 4 | 3 |

**Table 2. Experiment Design for Mix mode chromatography (MMC)**

| **Step** | **Buffer** | **Residence Time (min)** | **Column Volume (CV)** |
|---|---|---|---|
| Storage buffer removal | WFI | 5 | 3 |
| Sanitization | 0.5 N Sodium Hydroxide | 5 | 3 |
| Charge | 20mM Sodium Phosphate (NaP) + 1M Sodium chloride (NaCl), pH 7.2 ± 0.2 | 5 | 3 |
| Equilibration | 20mM Sodium Phosphate (NaP) pH 7.2 ± 0.2 | 5 | 7 - 10 |
| Load | Neutralized Protein A Eluate | 5 | Till loading volume |
| Wash 1 | 20mM Sodium Phosphate (NaP), pH 7.2 ± 0.2 | 5 | 5 |
| Wash 2 | 20mM Sodium Phosphate (NaP) + 60mM Arginine HCl, pH 7.2 ± 0.2 | 5 | 20%B step gradient in 10 CV |
| Elution | 20mM Sodium Phosphate (NaP) + 0.3M Arginine HCl, pH 7.2 ± 0.2 | 5 | 20 - 80%B linear in 10 CV |
| Regeneration | 20mM Sodium Phosphate (NaP) + 1M Sodium chloride (NaCl) | 5 | 5 |
| Sanitization | 0.5N Sodium Hydroxide | 5 | 3 |
| Storage | 0.1N Sodium Hydroxide | 5 | 3 |

### SE-HPLC Analysis of MMC OP (5L Batch)

| **Step outputs** | **Main Peak (%)** | **HMW (%)** | **Pre-peak (%)** |
|---|---|---|---|
| NPEL | 61.4 | 18.6 | 20 |
| MMC OP | 99.9 | 0.1 | Not Detected |

It is evident that Main peak purity is improved and HMW is reduced by 99.46% and pre-peak is not detectable.

### CE-SDS analysis of LMW in MMC OP

| **Process Step output** | **Total LMW (%)** |
|---|---|
| NPEL | 30.7 |
| MMC OP | 3.1 |

It is evident that LMW is reduced by 89.90%.

### Example 2 - Purification of Fc-fusion protein by performing Affinity Chromatography followed by MMC (Mix mode Chromatography) followed by AEX (Anion exchange chromatography).

All chromatographic processes were carried out using an AKTA Pure 150 system from Cytiva (formally known as GE Healthcare). Concentration of protein samples were determined by measuring absorbance at 280nm using Shimadzu Spectrophotometer. Protein A Chromatography resin (Mab Select Sure LX) obtained from Cytiva, MMC resin (Nuvia aPrime 4A) obtained from Bio-rad, AEX (Poros XQ). All Chemicals were obtained from JTB or Merck Millipore and were of GMP grade.

A Fc-fusion protein expressed in Chinese Hamster Ovary (CHO) cell line is captured using Protein A resin (Mab Select Sure LX, Cytiva) packed in XK 50/30 column. Eluted protein is further purified using Mix Mode chromatography resin (Nuvia aPrime 4A, BioRad) packed in XK 50/30 followed by Anion exchange chromatography resin (Poros XQ, ThermoFisher) packed in VL 32/250 column.

Fc-fusion protein is obtained from the suitable mammalian expression system comprising Fc-fusion protein which is CTLA4-IgG1. Fc-fusion protein present in the sample is loaded onto a Protein A chromatography resin and eluting the Fc-fusion protein from the Protein A resin for which experiment design is shown in table 1, wherein the eluted product was neutralized and referred to as Neutralized Protein A Elute (NPEL). The residence time is 4 min for all the phases.

Binding the NPEL to a mixed mode chromatography resin (MMC) and eluting the sample from the MMC resin by increasing arginine concentration for which experiment design is shown in table 2 referred to as Mix mode chromatography output (MMC OP), the residence time is 5 min for all the phases.

MMC OP further incubated with 0.5%. Triton X 100 for 1 hour and Anion Exchange chromatography (AEX) input was prepared by diluting with WFI.

Binding the AEX input to an anion exchange chromatography resin (AEX) and eluting sample from the AEX resin by increasing salt concentration for which the experiment design is shown in table 3 referred to as anion exchange chromatography output (AEX OP), the residence time is 4 min for all the phases.

**Table 3. Experiment design for Anion exchange chromatography**

| **Step** | **Buffer** | **Residence Time(min)** | **Column Volume (CV)** |
|---|---|---|---|
| Storage buffer removal | WFI | 4 | 3 |
| Sanitization | 0.5 N Sodium Hydroxide | 4 | 3 |
| Charge | 20mM Sodium Phosphate (NaP) + 1 M Sodium chloride (NaCl), pH 7.2 ± 0.2 | 4 | 3 |
| Equilibration | 20mM Sodium phosphate (NaP), pH 7.2 ± 0.2 | 4 | 10 |
| Load | MMC OP is diluted with WFI in a ratio of 1: 1.33, pH 7.2 ± 0.2 | 4 | Till loading volume |
| Wash 1 | 20mM Sodium phosphate (NaP), pH 7.2 ± 0.2 | 4 | 10 |
| Wash 2 | 20mM Sodium Phosphate (NaP) + 60mM Sodium chloride (NaCl), pH 7.2 ± 0.2 | 4 | 20%B step gradient in 5 CV |
| Elution | 20mM Sodium Phosphate (NaP) + 300 mM Sodium chloride (NaCl), pH 7.2 ± 0.2 | 4 | 20-70%B linear in 15 CV |
| Regeneration | 20mM Sodium Phosphate (NaP) + | 4 | 5 |
| | 1 M Sodium chloride (NaCl), pH 7.2 ± 0.2 | | |
| Sanitization | 0.5N Sodium Hydroxide | 4 | 3 |
| Storage | 0.1N Sodium Hydroxide | 4 | 3 |

### SE-HPLC Analysis of AEX OP (5L Batch)

| **Step outputs** | **Main Peak (%)** | **HMW (%)** | **Pre-peak (%)** |
|---|---|---|---|
| NPEL | 61.4 | 18.6 | 20 |
| MMC OP | 99.9 | 0.1 | Not Detected |
| AEX OP | 99.5 | 0.5 | Not Detected |

It is evident that Main peak purity is improved and HMW is reduced by 97.31% and pre-peak is not detectable.

### CE-SDS analysis of LMW in AEX OP (5L Batch)

| **Process Step output** | **Total LMW (%)** |
|---|---|
| NPEL | 30.7 |
| MMC OP | 3.1 |
| AEX OP | 2.1 |

It is evident that LMW is reduced by 93.16%.

### Example 3 - Purification of Fc-fusion by performing Affinity Chromatography followed by MMC (Mix mode Chromatography), AEX (Anion exchange chromatography) and HIC (Hydrophobic interaction chromatography) chromatography (5L scale with Triton).

All chromatographic processes were carried out using an AKTA Pure 150 system from Cytiva (formally known as GE Healthcare). Concentration of protein samples were determined by measuring absorbance at 280nm using Shimadzu Spectrophotometer. Protein A Chromatography resin (Mab Select Sure LX) obtained from Cytiva, MMC resin (Nuvia aPrime 4A) obtained from Bio-rad, AEX (Poros XQ), and HIC (Poros Benzyl) resins are obtained from ThermoFisher. Column hardwares of VL 32/250 and VL44/250 were obtained from Millipore, XK 50/30 columns were obtained from Cytiva. Pellicone 3 30kDa D screen cassettes and 30 kDa Centricon were obtained from Merck Millipore. 200 cm² TFF cassette holder obtained from Sartorius. All Chemicals were obtained from JTB or Merck Millipore and were of GMP grade.

A Fc-fusion protein expressed in Chinese Hamster Ovary (CHO) cell line is captured using Protein A resin (Mab Select Sure LX, Cytiva) packed in XK 50/30 column. Eluted protein is further purified using Mix Mode chromatography resin (Nuvia aPrime 4A, BioRad) packed in XK 50/30 followed by Anion exchange chromatography resin (Poros XQ, ThermoFisher) packed in VL 32/250 column. Final polishing was performed by Hydrophobic interaction chromatography resin (Poros Benzyl, ThermoFisher) packed in VL 44/250 column.

Fc-fusion protein is obtained from the suitable mammalian expression system comprising Fc-fusion protein which is CTLA4-IgG1. Fc-fusion protein present in the sample loaded to a Protein A chromatography resin and eluting the Fc-fusion protein from the Protein A resin for which experiment design is shown in table 1, wherein the eluted product was neutralized and referred to as Neutralized Protein A Elute (NPEL). The residence time is 4 min for all the phases.

Binding the protein mixture/sample or NPEL to a mixed mode chromatography resin (MMC) and eluting the sample from the MMC resin by increasing arginine concentration for which experiment design is shown in table 2 referred to as Mix mode chromatography output (MMC OP), the residence time is 5 min for all the phases.

MMC OP further incubated with 0.5%. Triton X 100 for 1 hour and Anion Exchange chromatography (AEX) input was prepared by diluting with WFI.

Binding the AEX input to an anion exchange chromatography resin (AEX) and eluting the sample from the AEX resin by increasing salt concentration for which the experiment design is shown in table 3 referred to as anion exchange chromatography output (AEX OP), the residence time is 4 min for all the phases.

AEX OP further diluted with HIC stock buffer to bind on a hydrophobic interaction chromatography resin (HIC) and eluting the protein mixture/sample from the HIC resin by decreasing salt concentration for which experiment design is shown in table 4 referred as hydrophobic interaction chromatography output (HIC OP), the residence time is 4 min for all the phases.

Ultrafiltration and Diafiltration-I (UFDF-I) is performed to concentrate HIC OP to approx. 20mg/mL and then buffer exchange into pre-formulation buffer until pH and conductivity of the diafiltrate reached to the same as pre-formulation buffer. Pellicone 3 30kDa screen cassettes from Millipore is used for Ultrafiltration-I and Diafiltration step. UFDF-I step for which experiment design is shown in table 5 referred to as Ultrafiltration & Diafiltration-I output (UFDF-I OP), Post diafiltration, UFDF-I OP further concentrated to target approx.150mg/mL protein concentration by using Millipore 30 kDa Centricon, referred to as ultrafiltration-II OP as shown in table 6. The results are further analyzed by SE-HPLC and CE-SDS.

**Table 4. Experiment design for Hydrophobic interaction chromatography**

| **Step** | **Buffer** | **Residence Time (min)** | **Column Volume (CV)** |
|---|---|---|---|
| Storage buffer removal | WFI | 4 | 3 |
| Sanitization | 0.5M Sodium Hydroxide | 4 | 3 |
| Equilibration | 20mM Sodium phosphate (NaP) + 0.4M Sodium Citrate, pH 7.2 ±0.2 | 4 | 5 |
| Load | AEX OP diluted with HIC Stock buffer (40mM Sodium Phosphate (NaP) + 0.8M Sodium Citrate, pH 7.2 ±0.2) in ratio of 1:1 | 4 | Till loading volume |
| Wash | 20mM Sodium Phosphate (NaP) + 0.4M Sodium Citrate, pH 7.2 ±0.2 | 4 | 3 |
| Elution | 20mM Sodium Phosphate (NaP) | 4 | 0-100%B linear gradient in 15 CV |
| Regeneration | WFI | 4 | 5 |
| Sanitization | 0.5N Sodium Hydroxide | 4 | 3 |
| Storage | 0.1N Sodium Hydroxide | 4 | 3 |

**Table 5. Experiment design for UFDF-I**

| **Step** | **Wash** | **Feed LMM** | **TMP (bar)** | **Remarks** |
|---|---|---|---|---|
| WFI | ~50L/m² | | | To remove storage solution |
| Sanitization | ~20L/m² | | | To clean the cassettes |
| WFI | ~100L/m² | | 0.8 to 1.2 | To remove CIP solution |
| Equilibration | ~10L/m² | 4-6 | | To conditioned membrane before loading |
| Load | Max. 300g/m² | | | Initial Conc. to initiate Diafiltration: ~20mg/mL |
| | | | | Diafiltration: NLT 8 DV |
| | | | | UFDF-I OP conc.: ~20mg/mL |
| Sanitization (0.5 N NaOH) | ~20L/m² | | | To clean the cassettes |
| WFI | ~50L/m² | | | To remove CIP solution |
| Storage (0.1 N NaOH) | ~20L/m² | | | Cassette storage |

**Table 6. Experiment design for UF-II**

| **Parameter** | **Value** |
|---|---|
| Centrifugation Force (RCF) | 4500 |
| Temperature | 20°C |
| Membrane Equilibration | 20mM Sodium Phosphate (NaP) + 0.2M Sodium Citrate |
| Ultrafiltration-II | Concentrate to ~ 150 mg/mL |

### SE-HPLC Analysis of UFDF OP II (5L Batch)

| **Step outputs** | **Main Peak (%)** | **HMW (%)** | **Pre-peak (%)** |
|---|---|---|---|
| NPEL | 61.4 | 18.6 | 20 |
| MMC OP | 99.9 | 0.1 | Not Detected |
| AEX OP | 99.5 | 0.5 | Not Detected |
| HIC OP | 99.9 | 0.1 | Not Detected |

| Virus filtration (Planova 20N) | | | |
|---|---|---|---|
| UFDF-II (VF OP) | 99.8 | 0.2 | Not Detected |

It is evident from the data that composition obtained from above mentioned four chromatographic purification steps has substantially reduced HMW, Pre-peak and purity of protein in UFDF-II is 99.8% analysed by SE-HPLC method.

### CE-SDS Analysis of UFDF OP II

| **Process Step output** | **Total LMW (%)** |
|---|---|
| NPEL | 30.7 |
| MMC OP | 3.1 |
| AEX OP | 2.1 |
| HIC OP | 0.3 |

| Virus filtration (Planova 20N) | |
|---|---|
| UFDF-II (VF OP) | 0.4 |

It is evident from the data that composition obtained from above mentioned four chromatographic purification steps has substantially reduced LMW from 30.7% to 0.4 % by analysed by CE-SDS method which is 98.70% reduction.

### Example 4 - Purification of Fc-fusion by performing Affinity Chromatography followed by MMC (Mix mode Chromatography), AEX (Anion exchange chromatography) and HIC (Hydrophobic interaction chromatography) chromatography (5L & at large scale 50L).

All chromatographic processes were carried out using an AKTA Pure 150 system from Cytiva (formally known as GE Healthcare). Concentration of protein samples were determined by measuring absorbance at 280nm using Shimadzu Spectrophotometer. Protein A Chromatography resin (Mab Select Sure LX) obtained from Cytiva, MMC resin (Nuvia aPrime 4A) obtained from Bio-rad, AEX (Poros XQ), and HIC (Poros Benzyl) resins are obtained from ThermoFisher. Column hardwares of VL 32/250 and VL44/250 were obtained from Millipore, XK 50/30 columns were obtained from Cytiva. Pellicone 3 30kDa D screen cassettes and 30 kDa Centricon were obtained from Merck Millipore. 200 cm² TFF cassette holder obtained from Sartorius. All Chemicals were obtained from JTB or Merck Millipore and were of GMP grade.

A Fc-fusion protein expressed in Chinese Hamster Ovary (CHO) cell line is captured using Protein A resin (Mab Select Sure LX, Cytiva) packed in XK 50/30 column. Eluted protein is further purified using Mix Mode chromatography resin (Nuvia aPrime 4A, BioRad) packed in XK 50/30 followed by Anion exchange chromatography resin (Poros XQ, ThermoFisher) packed in VL 32/250 column. Final polishing was performed by Hydrophobic interaction chromatography resin (Poros Benzyl, ThermoFisher) packed in VL 44/250 column.

Fc-fusion protein is obtained from the suitable mammalian expression system.

Harvested Fc-fusion protein is loaded onto a Protein A chromatography resin and eluting the Fc-fusion protein from the Protein A resin for which experiment design is shown in table 1, wherein the eluted product was neutralized and referred to as Neutralized Protein A Elute (NPEL). The residence time is 4 min for all the phases.

Binding the NPEL to a mixed mode chromatography resin (MMC) and eluting the sample/protein mixture from the MMC resin by increasing arginine concentration for which experiment design is shown in table 2 referred to as Mix mode chromatography output (MMC OP), the residence time is 5 min for all the phases.

Optionally, MMC OP may further incubated with 0.5%. Triton X 100 for 1hour and Anion Exchange chromatography (AEX) input was prepared by diluting with WFI.

Binding the AEX input to an anion exchange chromatography resin (AEX) and eluting the sample from the AEX resin by increasing salt concentration, for which the experiment design is shown in table 3 referred to as anion exchange chromatography output (AEX OP), the residence time is 4 min for all the phases.

AEX OP further diluted with HIC stock buffer to bind on a hydrophobic interaction chromatography resin (HIC) and eluting the sample from the HIC resin by decreasing salt concentration for which the experiment design is shown in table 4 and referred as hydrophobic interaction chromatography output (HIC OP), the residence time is 4 min for all the phases.

Ultrafiltration and Diafiltration-I (UFDF-I) is performed to concentrate HIC OP to approx. 20mg/mL and then buffer exchanged into pre-formulation buffer until pH and conductivity of the diafiltrate reached to the same as pre-formulation buffer. Pellicone 3 30kDa screen cassettes from Millipore will be used for Ultrafiltration-I and Diafiltration step. UFDF-I step for which experiment design is shown in table 5 and referred to as Ultrafiltration & Diafiltration-I output (UFDF-I OP). Post UFDF-I, UFDF-I OP further concentrated to target approx.150mg/mL protein concentration by using Millipore 30 kDa Centricon, referred to as ultrafiltration-II OP as shown in table 6. The experiment design which is further analyzed by SE-HPLC and CE-SDS.

### SE-HPLC Analysis of Process Intermediates (50L Batch)

| **Step outputs** | **Main Peak (%)** | **HMW (%)** | **Pre peak (%)** |
|---|---|---|---|
| NPEL | 65.3 | 17.8 | 16.9 |
| MMC OP | 98.8 | 0.2 | 1 |
| AEX OP | 99.1 | 0.3 | 0.6 |
| HIC OP | 99.5 | 0.5 | 0.0 |
| UFDF-II OP | 99.9 | 0.1 | 0.0 |

It is evident from the data that composition obtained from above mentioned four chromatographic purification steps has substantially reduced HMW, Pre-peak and purity of protein is 99.9% analysed by SE HPLC method.

### CE-SDS Analysis of Process Intermediates (50L Batch)

| **Step outputs** | **Total LMW (%)** |
|---|---|
| NPEL | 27.8 |
| MMC OP | 3.4 |
| AEX OP | 1.6 |
| HIC OP | 0.2 |
| UFDF-II OP | 0.3 |

It is evident from the data that composition obtained from above mentioned four chromatographic purification steps has substantially reduced LMW from 27.8% to 0.3 % by analysed by CE SDS method which is 98.92% reduction.

### N-Glycan analysis of DS (Drug substance)

| Quality Attributes | 50 L Scale | | |
|---|---|---|---|
| | NPEL | DS | |
| N-Glycan | High Mannose | 0.81% | 0.19% |
| | Afucosylation | 1.15% | 0.87% |
| | Total Sialyation | 53.07% | 53.84% |

### Example 5 - Purification of Fc-fusion by performing Affinity Chromatography followed by MMC (Mix mode Chromatography), AEX (Anion exchange chromatography) and HIC (Hydrophobic interaction chromatography) chromatography (5L Scale).

All chromatographic processes were carried out using an AKTA Pure 150 system from Cytiva (formally known as GE Healthcare). Concentration of protein samples were determined by measuring absorbance at 280nm using Shimadzu Spectrophotometer. Protein A Chromatography resin (Mab Select Sure LX) obtained from Cytiva, MMC resin (Nuvia aPrime 4A) obtained from Bio-rad, AEX (Poros XQ), and HIC (Poros Benzyl) resins are obtained from Thermofisher.

Column hardwares of VL 11/25 was obtained from Millipore, Highscale 10/40, XK-16/20 and Tricon 5/20 columns were obtained from Cytiva. Pellicone 3 cassette and 10kDa Amicon were obtained from Millipore. 200 cm² TFF cassette holder obtained from sartorius, and refrigerated centrifuge obtained from Beckman coulter. All Chemicals were obtained from JTB or Merck Millipore and were of GMP grade.

A Fc-fusion protein expressed in Chinese Hamster Ovary (CHO) cell line is captured using Protein A resin (Mab Select Sure LX, GE Healthcare) packed in VL 11/25 column. Eluted protein is further purified using Mix mode chromatography resin (Nuvia aPrime 4A, Bio Red) packed in XK-16/20 followed by Anion exchange chromatography resin (Poros XQ, Thermofisher) packed in Highscale 10/40 column. Final polishing was performed by Hydrophobic interaction chromatography resin (Poros Benzyl, Thermofisher) packed in Tricon 5/20 column.

Fc-fusion protein is obtained from the suitable mammalian expression system comprising Fc-fusion protein which is CTLA4-IgG1.

Fc-fusion protein present in the sample loaded to a Protein A chromatography resin and eluting the Fc-fusion protein from the Protein A resin for which experiment design is shown in table 7, wherein the eluted product was neutralized and referred to as Neutralized Protein A Elute (NPEL). The residence time is 4 min for all the phases.

Binding the NPEL to a mixed mode chromatography resin (MMC) and eluting the sample/protein mixture from the MMC resin by increasing arginine concentration for which experiment design is shown in table 8 referred to as Mix mode chromatography output (MMC OP), The residence time is 4 min for all the phases.

MMC OP further concentrate, and buffer exchanged in a suitable buffer with Pellicon 3 cassette with D-screen for which experiment design is shown in table 9, referred to as ultrafiltration and diafiltration-I output (UFDF-I OP).

Binding the UFDF-I OP to an anion exchange chromatography resin (AEX) and eluting the sample from the AEX resin for which experiment design is shown in table 10 referred to as anion exchange chromatography output (AEX OP), The residence time is 4 min for all the phases.

Binding the AEX OP to a hydrophobic interaction chromatography resin (HIC) and eluting the sample from the HIC resin for which experiment design is shown in table 11 referred to as hydrophobic interaction chromatography output (HIC OP), The residence time is 4 min for all the phases.

Ultrafiltration and diafiltration of HIC OP in a suitable stable buffer /formulation buffer with Millipore 10 kDa centricon, which is used for final concentration/formulation of commercial therapeutic proteins referred to as ultrafiltration and diafiltration chromatography output-II (UDFD-II OP) is shown table 12, further analysis done by SE-HPLC and CE-SDS is shown below.

**Table 7. Experiment Design for Affinity chromatography**

| **Step** | **Buffer** | **Residence Time (min)** | **Column Volume (CV)** |
|---|---|---|---|
| Storage buffer removal | WFI | 4 | 2-3 |
| Sanitization | 0.1 M NaOH | 4 | 2-3 |
| Equilibration | 20mM Tris HCl +150mM NaCl, pH 7.0±0.2 | 4 | 4-6 |
| Load | Clarified Harvest | 4 | Till loading volume |
| Wash 1 | 20mM Tris HCl +150mM NaCl, pH 7.0±0.2 | 4 | 3-5 |
| Wash 2 | 20mM Tris HCl +1M NaCl, pH 7.0±0.2 | 4 | 3-5 |
| Wash 3 | 20mM Tris HCl, pH 7.0±0.2 | 4 | 5-7 |
| Elution | 100mM Tris Acetate, pH 3.5±0.2 | 4 | 2-3 |
| Neutralization wash | 20mM Tris HCl, pH 7.0±0.2 | 4 | 3-5 |
| Sanitization | 0.1 M NaOH | 4 | 2-3 |
| Neutralization wash | 20mM Tris HCl, pH 7.0±0.2 | 4 | 3-5 |
| Storage | 2% Benzyl Alcohol in Wash 3 | 4 | 2-3 |

**Table 8. Experiment Design for Mix mode chromatography**

| **Step** | **Buffer** | **Residence Time (min)** | **Column Volume (CV)** |
|---|---|---|---|
| Storage buffer removal | WFI | 4 | 2-3 |
| Sanitization | 0.5N Sodium Hydroxide | 4 | 2-3 |
| Equilibration | 50 mM Tris HCl+20 mM Sodium Citrate, pH 7.0 ±0.2 | 4 | 4-6 |
| Load | NPEL Sample with + 20 mM Sodium Citrate, pH 7.0 ±0.2 | 4 | Till loading volume |
| Wash | 50 mM Tris HCl+20 mM Sodium Citrate, pH 7.0 ±0.2 | 4 | 3-5 |
| Elution | 50mM Tris HCl+0.4M Arginine HCl, pH 7.0±0.2 | 4 | 0-30%B linear followed by 30%B hold till 40% loaded protein gets eluted |
| Sanitization | 0.5N Sodium Hydroxide | 4 | 3-5 |
| Storage | 0.1N Sodium Hydroxide | 4 | 3-5 |

**Table 9. Experiment design for UFDF-I**

| **Parameter** | **Value** |
|---|---|
| Cross Flow | 360 LMH |
| Membrane Area | 176 cm2 |
| TMP | < 1 bar |
| Membrane Sanitization | 0.5L of 0.5M Sodium hydroxide solution |
| Membrane Equilibration | 50mM Tris HCl+ 10 mM Sodium Citrate + 0.2M Arginine, pH 7.0±0.2 |
| Ultrafiltration & Diafiltration | Concentrate MMC OP to 3-5 times of starting volume |
| | Buffer exchanged concentrated MMC OP in to 25mM Sodium phosphate, pH 7.0±0.2, Total DV: 6-8 DV |
| Membrane Sanitization | 0.5L of 0.5M Sodium hydroxide solution |
| Storage | 0.1 M Sodium hydroxide |

**Table 10. Experiment design for Anion exchange chromatography**

| **Step** | **Buffer** | **Residence Time (min)** | **Column Volume (CV)** |
|---|---|---|---|
| Sanitization | 0.5N Sodium Hydroxide | 4 | 2-3 |
| Equilibration | 25mM Sodium phosphate, pH 7.0±0.2 | 4 | 4-6 |
| Load | MMC OP in 25mM Sodium phosphate, pH 7.0±0.2 | 4 | Till loading volume |
| Wash | 25mM Sodium phosphate, pH 7.0±0.2 | 4 | 4 |
| Elution | 25mM Sodium phosphate+1M NaCl, pH 7.0±0.2 | 4 | 9%B Step gradient followed by 9-30%B in 20CV |
| Sanitization | 0.5M Sodium Hydroxide | 4 | 3-5 |
| Storage | 0.1M Sodium Hydroxide | 4 | 3-5 |

**Table 11. Experiment design for Hydrophobic interaction chromatography**

| **Step** | **Buffer** | **Residence Time (min)** | **Column Volume (CV)** |
|---|---|---|---|
| Stock buffer for dilution | 40mM Sodium Phosphate + 0.8M Sodium Citrate, pH 7.0 ±0.2 | NA | NA |
| Sanitization | 0.5M Sodium Hydroxide | 4 | 2-3 |
| Equilibration | 20mM Sodium phosphate+0.4M Sodium Citrate, pH 7.0 ±0.2 | 4 | 4-6 |
| Load | AEX OP in 20mM Sodium phosphate+0.4M Sodium Citrate, pH 7.0 ±0.2 | 4 | Till loading volume |
| Wash | 20mM Sodium phosphate+0.4M Sodium Citrate, pH 7.0 ±0.2 | 4 | 3-5 |
| Elution | 10mM Sodium Phosphate | 4 | 20%B for 2CV followed by 20-80%B in 13 CV |
| Regeneration | WFI | | 4-6 |
| Sanitization | 0.5M Sodium Hydroxide | 4 | 3-5 |
| Storage | 0.1M Sodium Hydroxide | 4 | 3-5 |

**Table 12. Experiment design for UDFD-II**

| **Parameter** | **Value** |
|---|---|
| Centrifugation Force (RCF) | 4500 |
| Temperature | 20°C |
| Membrane Equilibration | 15mM Sodium phosphate+0.2M Sodium Citrate, pH 7.0 ±0.2 |
| Ultrafiltration | 5-10 times of starting volume |
| Diafiltration | 6-8 DV in stable buffer |

### SEC-HPLC Analysis of Process Intermediates

| Process outputs | Main Peak (%) | HMW (%) | Pre-peak (%) |
|---|---|---|---|
| NPEL | 66.81 | 17.01 | 16.1 |
| MMC OP | 99.56 | 0.32 | 0 |
| UFDF-I OP | 99.70 | 0.26 | 0 |
| AEX OP | 99.81 | 0.16 | 0 |
| HIC OP | 99.61 | 0.39 | 0 |
| UFDF-II OP | 99.82 | 0.09 | 0 |

It is evident from the data that composition obtained from above mentioned four chromatographic purification steps has substantially reduced HMW, Pre-peak and purity of protein in UFDF-II OP is 99.82% analysed by SE HPLC method.

### Size related Variants: CE-SDS

| Process Step output | Total LMW |
|---|---|
| NPEL | 29.9 |
| MMC OP | 3.86 |
| UFDF-I OP | 4.22 |
| AEX OP | 2.05 |
| HIC OP | 1.19 |
| UFDF-II OP | 1.56 |

It is evident from the data that composition obtained from above mentioned four chromatographic purification steps has substantially reduced LMW from 29.9% to 1.56% by analysed by CE-SDS method which is about 95% reduction.

### Summary Paragraphs:

1. A process for purification of IgG1 containing protein comprising:
   a) collecting the IgG1 containing protein from the suitable mammalian expression system and the impurities;
   b) contacting the IgG1 containing protein on to a affinity chromatography;
   c) eluting the IgG1 containing protein;
   d) contacting the eluted IgG1 containing protein onto mixed-mode chromatography, optionally followed by other suitable purification steps.
2. The process as disclosed in paragraph 1, wherein the mixed-mode chromatography is performed in bind-elute mode.
3. The process as disclosed in paragraph 2, wherein the bind-elute mode comprises: (i) loading the eluted IgG1 protein from Affinity chromatography at suitable pH and/or conductivity for binding (ii) performing washing at suitable pH and/or conductivity (iii) eluting the IgG1 protein at suitable pH and/or conductivity.
4. The process as disclosed in paragraph 3, wherein the loading is performed at suitable pH selected from about 6.5 to about 7.5.
5. The process as disclosed in paragraph 3, wherein the loading is performed at suitable conductivity selected from about 3 mS/cm to about 5 mS/cm.
6. The process as disclosed in paragraph 3, wherein the suitable pH and/or conductivity is maintained with suitable buffer selected from sodium phosphate, tris-HCl, sodium citrate, and arginine HCl.
7. The process as disclosed in paragraph 3, wherein the suitable washing comprises:
   (i) first wash with equilibration buffer at suitable pH 7.2±0.2 and/or conductivity 2.7±0.3;
   (ii) second wash at the same pH as of the first wash buffer and/or a conductivity higher than the first wash buffer.
8. The process as disclosed in paragraph 7, wherein the second wash comprises conductivity selected from about 5 mS/cm to about 25 mS/cm, preferably 7.2±0.5.
9. The process as disclosed in paragraph 7, wherein the second wash buffer comprises sodium phosphate and optionally suitable additive preferably arginine HCl.
10. The process as disclosed in paragraph 3, wherein the elution is performed at suitable pH 7.2±0.2 and conductivity 21.0±3.0 mS/cm.
11. The process as disclosed in paragraph 3, wherein the elution is performed with buffer sodium phosphate and suitable additive preferably arginine HCl.
12. The process as disclosed in paragraph 11, wherein the elution is performed in linear gradient wherein the concentration of elution buffer increased from 0 to about 80%, preferably 0 to about 30%, more preferably about 20 to about 80%.
13. The process as disclosed in paragraph 11, wherein the elution starts at an ascending value of about 0.5AU/cm and ends at descending value of about 1.5 AU/cm .
14. The process as disclosed in paragraph 1, wherein the mixed-mode chromatography is selected from Nuvia aPrime 4A, Capto adhere ImpRes, Capto adhere (N-Benzyl-N-methyl ethanol amine as ligand), Capto MMC (MMC ligand), MEP Hypercel (4-marcaptomethyl pyridine as ligand), HEA Hypercel (hexyl amine as ligand), and PPA Hypercel (phenylpropylamine as ligand).
15. The process as disclosed in paragraph 1, wherein the Affinity chromatography is selected from Protein A, Protein G, and Protein L.
16. The process as disclosed in paragraph 1, wherein the eluted IgG1 containing protein has reduced impurities selected from Pre-Peak, HMWs and LMWs.
17. The process as disclosed in paragraph 16, wherein the pre-peak and/or HMWs and/or LMWs reduced by more than 95%, preferably more than 98%.
18. The process as disclosed in paragraph 1, wherein the other purification method is selected from ion exchange chromatography, hydrophobic interaction chromatography, ultrafiltration and diafiltration.
19. The process as disclosed in paragraph 18, wherein the ion exchange chromatography selected from anion exchange and cation exchange chromatography.
20. The process as disclosed in paragraph 19, wherein the anion exchange chromatography resins are selected from Poros XQ, Poros HQ DEAE, Sepharose fast flow, Fractogel^{®} EMD DEAE (M), Toyopearl DEAE-650, Toyopearl DEAE-650, and Nuvia Q.
21. The process as disclosed in paragraph 20, wherein the anion exchange is strong anion exchange.
22. The process as disclosed in paragraph 19, wherein the anion exchange chromatography reduces undesired glycans selected from high mannose and afucosylated glycanss.
23. The process as disclosed in paragraph 22, wherein the anion exchange reduces undesired glycan selected from high mannose by less than 50%, preferably less than 77% and afucosylated glycans by less than 50%, preferably less than 25%.
24. The process as disclosed in paragraph 19, wherein the anion exchange is performed in bind elute mode comprising: (i) loading the eluted IgG1 protein at suitable pH and/or conductivity for binding (ii) performing washing at suitable pH and/or conductivity (iii) eluting the IgG1 protein at suitable pH and/or conductivity.
25. The process as disclosed in paragraph 24, wherein the loading is performed at suitable pH selected from about 6 to about 8 and conductivity selected from about 5 mS/cm to about 8 mS/cm.
26. The process as disclosed in paragraph 24, wherein the suitable pH and/or conductivity is maintained with suitable buffer selected from sodium phosphate, Tris-HCl, sodium chloride.
27. The process as disclosed in paragraph 24, wherein the suitable washing comprises:
   (i) first wash with equilibration buffer at suitable pH 7.2±0.2 and/or conductivity 2.7±0.3 mS/cm;
   (ii) second wash at the same pH as of the first wash buffer and/or a conductivity higher than the first wash buffer selected from about 5mS/cm to about 20mS/cm.
28. The process as disclosed in paragraph 27, wherein the second wash buffer is selected from sodium phosphate and sodium chloride.
29. The process as disclosed in paragraph 24, wherein the elution is performed at suitable pH 7.2±0.2 and conductivity 30.0±3.0 mS/cm.
30. The process as disclosed in paragraph 24, wherein the elution is performed with buffer is selected from sodium phosphate and sodium chloride.
31. The process as disclosed in paragraph 25, wherein the elution is performed in linear gradient, wherein the concentration of elution buffer is increased from about 20% to about 80%, preferably about 20% to about 70%.
32. The process as disclosed in paragraph 31, wherein the elution starts at an ascending value of about 10mAU/cm to about descending 80mAU/cm.
33. The process as disclosed in paragraph 18, wherein the hydrophobic interaction chromatography is selected from Poros Benzyl, Butyl Toyopearl 650 M resin, Toyopearl Phenyl-650, Butyl Sepharose 6 Fast Flow, Phenyl Sepharose 6 Fast Flow, Butyl Sepharose HP, Phenyl Sepharose 6 Fast Flow high sub, Capto Phenyl high sub, and Capto Butyl impRes.
34. The process as disclosed in paragraph 33, wherein the hydrophobic interaction chromatography is performed in bind-elute mode.
35. The process as disclosed in paragraph 33, wherein the hydrophobic interaction chromatography reduces impurity selected from Prepeak, HMWs, and LMWs by less than 50%, preferably less than 70%.
36. The process as disclosed in paragraph 34, wherein the bind-elute mode comprises (i) loading the eluted IgG1 protein at suitable pH and/or conductivity for binding (ii) performing washing at suitable pH and/or conductivity (iii) eluting the IgG1 protein at suitable pH and/or conductivity.
37. The process as disclosed in paragraph 36, wherein the loading is performed at suitable pH selected from about 6 to 8 and suitable conductivity selected from about 40mS/cm to about 70 mS/cm, preferably 50±0.2 mS/cm.
38. The process as disclosed in paragraph 36, wherein the suitable pH and/or conductivity is maintained with suitable buffer selected from sodium phosphate and sodium citrate.
39. The process as disclosed in paragraph 36, wherein the suitable washing is performed with buffer selected from sodium phosphate, sodium citrate at pH 7.2±0.2 and conductivity selected from about 35 mS/cm to about 50 mS/cm.
40. The process as disclosed in paragraph 36, wherein the elution is performed with suitable buffer selected from sodium phosphate, sodium citrate at suitable pH 7.2±0.2 and conductivity 2.7±0.3 mS/cm.
41. The process as disclosed in paragraph 40, wherein the elution is performed in linear gradient.
42. The process as disclosed in paragraph 41, wherein the elution starts at an ascending value of about 1.85AU/cm and ends at descending value of about 2.0 AU/cm.
43. A process for purification of IgG1 containing protein comprising:
   a) obtaining IgG1 protein mixture from the suitable mammalian expression system comprising protein of interest and impurities selected from pre-peak, HMWs, LMWs, and undesired glycan;
   b) loading onto Protein A affinity chromatography;
   c) eluting from Protein A affinity chromatography;
   d) loading the elute onto a Mixed-mode chromatography;
   e) eluting from Mixed-mode chromatography;
   f) loading the elute onto an anion exchange chromatography;
   g) eluting from anion exchange chromatography;
   h) loading the elute onto a Hydrophobic interaction chromatography;
   i) eluting from a Hydrophobic interaction chromatography;
   wherein the eluted protein from Hydrophobic interaction chromatography is optionally followed by other suitable purification steps; wherein the elute comprises substantially higher purity of protein of interest and reduce amount of said impurities.
44. The process as disclosed in paragraph 43, reduces more than 95% of the impurity selected from pre-peak, HMWs, and LMWs.
45. The process as disclosed in paragraph 43, reduces more than 98% of the impurity selected from pre-peak, HMW, and LMW.
46. The process as disclosed in paragraph 43, wherein the steps (b) to (i) carried out with same buffer at same pH 7.2±0.2.
47. The process as disclosed in paragraph 43, wherein the other purification method is selected from ultrafiltration and/or diafiltration is performed at least one or two time.
48. The process as disclosed in paragraph 1 or paragraph 43, wherein the IgG1 containing protein is antibody or fusion protein wherein fusion protein is selected from CTLA4-IgG1 or CTLA4-Fc, TNFR-Fc, VEGF-Fc or wherein antibody is selected from Rituximab, Palivizumab, Infliximab, Trastuzumab, Alemtuzumab, Adalimumab, Ibritumomab tiuxetan, Omalizumab, Cetuximab ,Bevacizumab, Natalizumab, Eculizumab, Certolizumab pegol, Ustekinumab, Canakinumab, Golimumab, Ofatumumab, Tocilizumab, Denosumab, Belimumab, Ipilimumab, Brentuximab vedotin, Pertuzumab, Trastuzumab emtansine, Raxibacumab, Obinutuzumab, Siltuximab, Ramucimmab, Vedolizumab, Blinatumomab, Nivolumab, Pembrolizumab, Darucizumab, Necitumumab, Dinutuximab, Secukinumab, Mepolizumab, Alirocumab, Evolocumab, Daratumumab, Elotuzumab, Ixekizumab, Reslizumab, Olaratumab, Bezlotoxumab, Atezolizumab, Obiltoxaximab, Sarilumab, Ocrelizumab, Tildrakizumab, Romosozumab, Brolucizumab, and Crizanlizumab.
49. The process as disclosed in paragraph 48, wherein the CTLA4-IgG1 or CTLA4-Fc is Abatacept or Belatacept.
50. A pharmaceutical composition of CTLA4-IgG1 or CTLA4-Fc fusion protein comprises substantially purified monomer of said fusion protein having purity at least 90% and HMWs less than about 0.2 %, measure by SE-HPLC.
51. A pharmaceutical composition of CTLA4-IgG1 or CTLA4-Fc fusion protein comprises substantially purified monomer of said fusion protein having purity at least 90% and pre-peak is less than about 0.2%, measure by SE-HPLC.
52. The pharmaceutical composition as disclosed in paragraph 50 or paragraph 51, wherein the monomer purity is selected from more than about 90% or about 91% or about 92% or about 93% or about 94% or about 95% or about 96% or about 97% or about 98% or about 99%.

## Claims

1. A process for purification of IgG1 containing protein comprising:
a) obtaining IgG1 protein mixture from the suitable mammalian expression system comprising protein of interest and impurities selected from pre-peak, HMWs, LMWs, and undesired glycan;
b) loading onto Protein A affinity chromatography;
c) eluting from Protein A affinity chromatography;
d) loading the elute onto a Mixed-mode chromatography;
e) eluting from Mixed-mode chromatography;
f) loading the elute onto an anion exchange chromatography;
g) eluting from anion exchange chromatography;
h) loading the elute onto a Hydrophobic interaction chromatography;
i) eluting from a Hydrophobic interaction chromatography;
wherein the eluted protein from Hydrophobic interaction chromatography is optionally followed by other suitable purification steps; wherein the elute comprises substantially higher purity of protein of interest and reduce amount of said impurities.

2. The process as claimed in claim 1, reduces more than 95% of the impurity selected from prepeak, HMWs, and LMWs.

3. The process as claimed in claim 1, reduces more than 98% of the impurity selected from prepeak, HMW, and LMW.

4. The process as claimed in claim 1, wherein the steps (b) to (i) carried out with same buffer at same pH 7.2±0.2.

5. The process as claimed in claim 1, wherein the other purification method is selected from ultrafiltration and/or diafiltration is performed at least one or two time.

6. The process as claimed in claim 1, wherein the IgG1 containing protein is antibody or fusion protein wherein fusion protein is selected from CTLA4-IgG1 or CTLA4-Fc, TNFR-Fc, VEGF-Fc or wherein antibody is selected from Rituximab, Palivizumab, Infliximab, Trastuzumab, Alemtuzumab, Adalimumab, Ibritumomab tiuxetan, Omalizumab, Cetuximab, Bevacizumab, Natalizumab, Eculizumab, Certolizumab pegol, Ustekinumab, Canakinumab, Golimumab, Ofatumumab, Tocilizumab, Denosumab, Belimumab, Ipilimumab, Brentuximab vedotin, Pertuzumab, Trastuzumab emtansine, Raxibacumab, Obinutuzumab, Siltuximab, Ramucimmab, Vedolizumab, Blinatumomab, Nivolumab, Pembrolizumab, Darucizumab, Necitumumab, Dinutuximab, Secukinumab, Mepolizumab, Alirocumab, Evolocumab, Daratumumab, Elotuzumab, Ixekizumab, Reslizumab, Olaratumab, Bezlotoxumab, Atezolizumab, Obiltoxaximab, Sarilumab, Ocrelizumab, Tildrakizumab, Romosozumab, Brolucizumab, and Crizanlizumab.

7. The process as claimed in claim 6, wherein the CTLA4-IgG1 or CTLA4-Fc is Abatacept or Belatacept.

8. The process as claimed in claim 1, wherein the eluted protein mixture obtained from Protein A affinity chromatography and/or mixed mode chromatography column is further incubated with detergent or surfactant for viral inactivation selected from about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 70 minutes, about 80 minutes.

9. The process as claimed in claim 8, wherein the detergent or surfactant is selected from Triton X100 or Octo X100, sugar-based detergent n-dodecyl-β-D-maltoside, preferably Triton X100

10. The process as claimed in claim 8, wherein the viral inactivated protein mixture subjected to filtration before loading onto anion exchange chromatography.

11. The process as claimed in claim 1, wherein the eluted IgG1 containing protein comprises a composition having HMW less than 0.2% and pre-peak below detection limit.

12. The process as claimed in claim 1, wherein the hydrophobic interaction chromatography resin is selected from Poros Benzyl, Butyl Toyopearl 650 M resin, Toyopearl Phenyl-650, Butyl Sepharose 6 Fast Flow, Phenyl Sepharose 6 Fast Flow, Butyl Sepharose HP, Phenyl Sepharose 6 Fast Flow high sub, Capto Phenyl high sub, Capto Butyl impRes.

13. The process as claimed in claim 1, wherein the loading onto the Hydrophobic interaction chromatography comprises elute of anion exchange chromatography mixed with Hydrophobic interaction chromatography (HIC) stock buffer in 1:1 dilution.

14. The process as claimed in claim 13, wherein mixing the elute of anion exchange chromatography with Hydrophobic interaction chromatography (HIC) stock buffer stops when final conductivity reaches to about from 40 mS/cm to about 70 mS/cm, preferably about 48 mS/cm to 52 mS/cm.

15. The process as claimed in claim 1, wherein the IC stock buffer comprises about 20 mM to about 50 mM, preferably 40 mM Sodium phosphate (NaP) and 0.6M to about 1M sodium citrate, preferably 0.8M sodium citrate wherein pH of the HIC stock buffer adjusted to 7.2±0.2 before loading and conductivity is selected from about 30 mS/cm to about 70 mS/cm, preferably 50±0.2 mS/cm.

16. The process as claimed in claim 1, wherein the hydrophobic interaction chromatography is performed in bind-elute mode.

17. The process as claimed in claim 1, wherein the protein is a Fc-fusion protein; wherein the process is free from at least one impurity selected from Host cell proteins (HCP) comprising:
a) collecting the first protein sample/mixture from the suitable mammalian expression system comprising the Fc-fusion protein and impurities;
b) contacting the protein sample/mixture to affinity chromatography column;
c) eluting the Fe-fusion protein from affinity chromatography column to form second protein mixture;
d) contacting the second protein mixture to mixed-mode chromatography column;
e) eluting the Fc-fusion protein from a mixed-mode chromatography column to form third protein mixture;
f) contacting the third protein mixture to anion exchange chromatography column;
g) eluting the Fc-fusion protein from anion exchange chromatography column to form fourth protein mixture;
h) contacting the fourth protein mixture to hydrophobic interaction chromatography column;
i) eluting the Fc-fusion protein from hydrophobic interaction chromatography column to form fifth protein mixture;
wherein the eluted Fc-fusion protein is substantially free of host cell proteins (HCP) impurities.

18. The process as claimed in claim 17, wherein the Fc-fusion protein comprises reduced HCP impurities at drug substance level after purification selected from about 10 ng/mg or less, about 9 ng/mg or less, about 8 ng/mg or less, about 7 ng/mg or less, about 6 ng/mg or less, about 5 ng/mg or less, about 4 ng/mg or less, about 3 ng/mg or less, about 2 ng/mg or less, about 1 ng/mg or less.

19. A pharmaceutical composition of CTLA4-IgG1 or CTLA4-Fc fusion protein comprises substantially purified monomer of said fusion protein having purity at least 90% and HMWs less than about 0.2%, measure by SE-HPLC, optionally wherein pre-peak is less than about 0.2%.

20. The pharmaceutical composition as claimed in claim 19 wherein the monomer purity is selected from more than about 90% or about 91% or about 92% or about 93% or about 94% or about 95% or about 96% or about 97% or about 98% or about 99%.
